# FASCICULE DE BREVET EUROPEEN

(11) **EP 2 207 525 B1**
(45) Date de publication et mention de la délivrance du brevet: **22.02.2017**
(21) Numéro de dépôt: 08841367.9
(22) Date de dépôt: 03.10.2008
(51) Int. Cl.: A61K 8/85, A61Q 1/04, A61Q 1/06, A61Q 3/02, C08G 63/48, A61K 8/72, A61K 47/30, A61P 17/00

(54) **COMPOSITION COSMETIQUE OU DERMATOLOGIQUE COMPRENANT UN POLYMERE PORTANT DES GROUPES DE JONCTION, ET PROCEDE DE TRAITEMENT COSMETIQUE**
KOSMETIK- ODER HAUTPFLEGEZUSAMMENSETZUNG MIT EINEM VERBINDUNGSGRUPPEN TRAGENDEN POLYMER SOWIE KOSMETISCHES BEHANDLUNGSVERFAHREN
COSMETIC OR DERMATOLOGICAL COMPOSITION COMPRISING A POLYMER BEARING JUNCTION GROUPS, AND COSMETIC TREATMENT METHOD

(30) Priorité: 05.10.2007 FR 0758099; 02.11.2007 US 984738 P
(43) Date de publication de la demande: 21.07.2010
(73) Titulaire: L'Oréal, 75008 Paris (FR)
(72) Inventeur: CHODOROWSKI-KIMMES, Sandrine, F-60300 Senlis (FR); GIUSTINIANI, Pascal, F-92250 La Garenne Colombes (FR)
(74) Mandataire: Dodin, Catherine
(86) Numéro de dépôt international: PCT/FR2008/051795
(87) Numéro de publication internationale: WO 2009/053594

(56) Documents cités:
- FR-A- 2 825 628
- FR-A- 2 879 920

## Description

La présente invention a trait à des compositions cosmétiques comprenant de nouveaux polymères de la famille des polycondensats, ainsi qu'aux procédés de traitement cosmétiques les employant.

Il existe de nombreuses compositions cosmétiques pour lesquelles des propriétés de brillance du film déposé, après application sur les matières kératiniques (peau, lèvres, phanères), sont souhaitées. On peut citer par exemple les rouges à lèvres, les vernis à ongles ou encore certains produits capillaires.

Afin d'obtenir un tel résultat, il est possible d'associer des matières premières particulières, notamment des lanolines, avec des huiles dites brillantes, telles que les polybutènes qui présentent toutefois une viscosité élevée; ou des esters d'acide ou d'alcool gras dont le nombre de carbone est élevé; ou bien certaines huiles végétales; ou encore des esters résultants de l'estérification partielle ou totale d'un composé aliphatique hydroxylé avec un acide aromatique, comme décrit dans la demande de brevet EP1097699.

Il est également connu d'associer des lanolines avec des polyesters obtenus par réaction séquencée de l'huile de ricin avec l'acide isostéarique puis avec l'acide succinique, tel que décrit dans le brevet US6342527.

Pour améliorer la brillance du film déposé, ainsi que sa tenue, il a également été proposé d'utiliser des esters résultant de la condensation d'un polyol avec un acide carboxylique de type "néo", notamment dans FR2838049.

On peut également citer EP1457201, qui décrit une composition associant un polyester de triglycérides d'acides carboxyliques hydroxylés et une huile de faible masse moléculaire choisie parmi les polybutylènes, les polyisobutylènes hydrogénés, les polydécènes hydrogénés ou non, les copolymères de vinylpyrrolidones, les esters d'acides gras linéaires, les esters hydroxylés, les esters d'alcools gras ou d'acides gras ramifiés en C24-C28, les huiles siliconées et/ou les huiles d'origine végétale.

Dans la demande de brevet EP1155687, on décrit un procédé consistant à incorporer, dans une phase huileuse constituée d'une huile cosmétiquement acceptable, un organopolysiloxane possédant au moins 2 groupes susceptibles d'établir des liaisons hydrogènes.

Toutefois, ces compositions et associations, même si elles peuvent améliorer la brillance, sont encore jugées insuffisantes dans une optique de longue tenue de cette brillance dans le temps. En effet, on a constaté que la tenue dans le temps de cette brillance était limitée.

Le but de la présente invention est de proposer des compositions cosmétiques qui contiennent de nouveaux polymères susceptibles d'allier la brillance à la longue tenue de la composition et de la brillance, tout en gardant bien évidemment les propriétés cosmétiques requises.

On recherche tout particulièrement des polymères susceptibles de conférer une brillance significative à un dépôt notamment filmogène, tout en maintenant une bonne durabilité dans le temps de cette brillance; ceci peut trouver une application particulièrement avantageuse dans le domaine des rouges à lèvres. On recherche également des polymères pouvant en outre conférer à la composition une excellente tenue dans le temps sur les matières kératiniques, notamment sur les lèvres.

Après d'importantes recherches, la demanderesse a découvert de façon surprenante et inattendue que certains polycondensats fonctionnalisés pouvaient conduire à des performances améliorées en terme de brillance, de maintien de ladite brillance, et en outre de longue tenue du film obtenu, tout en étant véhiculables dans les milieux cosmétiques usuels, notamment les milieux huileux cosmétiques usuels.

La présente invention a donc pour objet une composition cosmétique ou dermatologique comprenant, dans un milieu cosmétiquement ou dermatologiquement acceptable, un polymère comportant :
(a) un squelette polymérique susceptible d'être obtenu par réaction :
   - de 10 à 60% en poids, par rapport au poids total dudit squelette, d'au moins un mélange d'acide monocarboxylique aromatique comprenant de 6 à 32 atomes de carbone et d'acide monocarboxylique non aromatique comprenant de 6 à 32 atomes de carbone ;
   - de 5 à 60% en poids, par rapport au poids total dudit squelette, d'au moins un acide polycarboxylique comprenant au moins 2 groupes carboxyliques COOH, et/ou un anhydride cyclique d'un tel acide polycarboxylique ;
      et
(b) au moins un groupe de jonction lié audit squelette polymérique et capable d'établir des liaisons H avec un ou plusieurs groupes de jonction partenaires, chaque appariement d'un groupe de jonction faisant intervenir au moins 3 liaisons H (hydrogène),
   ledit squelette étant lié au groupe de jonction par au moins une fonction uréthanne,
   le groupe de jonction porteurs de groupes isocyanates étant de formule OCN-A-NCO, le radical divalent A étant de structure : 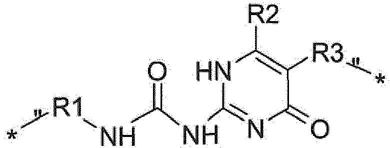 dans laquelle:
   - R1 et R3, identiques ou différents, représentent un radical carboné divalent choisi parmi (i) un groupe alkyle linéaire ou ramifié en C₁-C₃₀, (ii) un groupe cycloalkyle en C₄-C₁₂ et (iii) un groupe aryle en C₄-C₁₂; comprenant éventuellement 1 à 8 hétéroatomes choisis parmi O N, S, F, Si et P; et/ou éventuellement substitué par une fonction ester, amide ou par un radical alkyle en C₁-C₁₂; ou un mélange de ces groupes;
   - R2 représente H, halogène (Br, Cl, F) ou un groupe hydrocarboné monovalent, linéaire, ramifié ou cyclique, en C1-C32, saturé ou non, éventuellement aromatique, pouvant contenir un ou plusieurs hétéroatomes choisis parmi O S, N, P, F, ou de formule B-NCO, le radical monovalent B étant de formule : 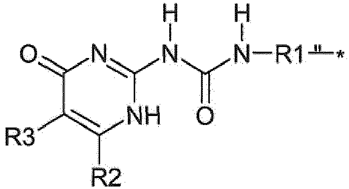 dans laquelle :
      - le radical R₁ représentant un groupe carboné divalent choisi parmi (i) un groupe alkyle linéaire ou ramifié en C₁-C₃₀, (ii) un groupe cycloalkyle en C₄-C₁₂ et (iii) un groupe aryle en C₄-C₁₂; comprenant éventuellement 1 à 8 hétéroatomes choisis parmi O N, S, F, Si et P; et/ou éventuellement substitué par une fonction ester, amide ou par un radical alkyle en C₁-C₁₂; ou un mélange de ces groupes;
      - les radicaux R2 et R3, identiques ou différents, représentent un atome d'hydrogène ou un radical carboné, notamment hydrocarboné (alkyle), linéaire, ramifié ou cyclique, saturé ou insaturé, en C1-C32, pouvant comprendre un ou plusieurs hétéroatomes choisis parmi O, N, S, F, Si et P.

Un autre objet de l'invention est un procédé de traitement cosmétique utilisant ladite composition.

Les compositions cosmétiques selon l'invention présentent une bonne applicabilité et une bonne couvrance; une bonne adhérence sur le support, que cela soit sur l'ongle, les cheveux, les cils, la peau ou les lèvres; une flexibilité et une résistance du film adéquates, ce qui permet d'éviter les craquelures, par exemple dans le cas des vernis ou des rouges à lèvres; ainsi qu'un excellent niveau de brillance durable.

Les propriétés de confort et de glissant sont également très satisfaisantes. Le film obtenu présente une rigidité et une souplesse adéquates pour son utilisation dans des compositions cosmétiques de type rouges à lèvres ou fonds de teint, tout en ayant une brillance et une tenue de la brillance telles que recherchées.

Par ailleurs, et de manière avantageuse et surprenante, ces polycondensats fonctionnalisés sont aisément véhiculables dans les milieux solvants ou huileux cosmétiques, notamment les huiles, les alcools gras et/ou les esters gras, ce qui facilite leur mise en oeuvre dans le domaine cosmétique, notamment dans les rouges à lèvres ou les fonds de teint.

Ils présentent une solubilité convenable dans des milieux huileux cosmétiques variés, tels que les huiles végétales, les alcanes, les esters qu'ils soient courts de type acétate de butyle ou d'éthyle, ou gras, les alcools gras, les huiles de silicone, et notamment comprenant l'isododécane, le Parléam, l'isononanoate d'isononyle, l'octyldodécanol, la phényl triméthicone, le benzoate d'alkyle en C12-C15 et/ou la D5 (décaméthylcyclopentasiloxane).

Les polycondensats fonctionnalisés selon l'invention sont avantageusement branchés (ramifiés); on peut penser que ceci permet de générer un réseau par enchevêtrement des chaînes polymériques, et donc d'obtenir les propriétés recherchées, notamment en terme de tenue améliorée, de brillance améliorée, et de solubilité.

Les polycondensats de type alkyde formant le squelette polymérique (appelé ci-après POL) sont susceptibles d'être obtenus par estérification/polycondensation, selon les méthodes connues de l'homme du métier, des constituants décrits ci-après.

L'un des constituants nécessaires pour la préparation de ces polycondensats est un composé comprenant 3 à 6 groupes hydroxyles (polyol), notamment 3 à 4 groupes hydroxyles. On peut bien évidemment utiliser un mélange de tels polyols. Ledit polyol peut notamment être un composé carboné, notamment hydrocarboné, linéaire, ramifié et/ou cyclique, saturé ou insaturé, comprenant 3 à 18 atomes de carbone, notamment 3 à 12, voire 4 à 10 atomes de carbone, et 3 à 6 groupes hydroxy (OH), et pouvant comprendre en outre un ou plusieurs atomes d'oxygène intercalés dans la chaîne (fonction éther). Ledit polyol est de préférence un composé hydrocarboné saturé, linéaire ou ramifié, comprenant 3 à 18 atomes de carbone, notamment 3 à 12, voire 4 à 10 atomes de carbone, et 3 à 6 groupes hydroxy (OH).

Il peut être choisi parmi, seul ou en mélange :
- les triols, tels que le 1,2,4-butanetriol, le 1,2,6-hexanetriol, le triméthyloléthane, le triméthylolpropane, le glycérol;
- les tétraols, tels que le pentaérythritol (tétraméthylolméthane), l'érythritol, le diglycérol ou le ditriméthylolpropane;
- les pentols tels que le xylitol,
- les hexols tels que le sorbitol et le mannitol; ou encore le dipentaérythritol ou le triglycérol.

De préférence, le polyol est choisi parmi le glycérol, le pentaérythritol, le sorbitol et leurs mélanges; et encore mieux est du pentaérythritol.

Le polyol, ou le mélange de polyol, représente de préférence 10 à 60% en poids, notamment 12 à 40% en poids, et mieux 14 à 25% en poids, du poids total du polycondensat formant le squelette polymérique.

Un autre constituant nécessaire pour la préparation de ces polycondensats est un mélange d'acides monocarboxyliques comprenant 6 à 32 atomes de carbone.

L'un des acides monocarboxylique est non aromatique. Il peut être saturé ou insaturé, linéaire, ramifié et/ou cyclique, comprenant 6 à 32 atomes de carbone, notamment 8 à 28 atomes de carbone et encore mieux 10 à 20, voire 12 à 18, atomes de carbone. Par acide monocarboxylique non aromatique, on entend un composé de formule RCOOH, dans laquelle R est un radical hydrocarboné saturé ou insaturé, linéaire, ramifié et/ou cyclique, comprenant 5 à 31 atomes de carbone, notamment 7 à 27 atomes de carbone, et encore mieux 9 à 19 atomes de carbone, voire 11 à 17 atomes de carbone. De préférence, le radical R est saturé. Encore mieux, ledit radical R est linéaire ou ramifié, et préférentiellement en C5-C31.

Les acides monocarboxyliques non aromatiques peuvent être choisis parmi les acides monocarboxyliques polyinsaturés conjugués, les acides monocarboxyliques non conjugués, et leur mélange. Les acides non conjugués comprennent les acides saturés, les acides monoinsaturés et les acides polyinsaturés non conjugués.

L'autre acide monocarboxylique est aromatique. Il peut comprendre de 7 à 11 atomes de carbone, éventuellement en outre substitué par 1 à 3 radicaux alkyles, saturés ou insaturés, linéaires, ramifiés et/ou cycliques, qui comprennent 1 à 32 atomes de carbone, notamment 2 à 12, voire 3 à 8 atomes de carbone. On peut bien évidemment utiliser un mélange de tels acides monocarboxyliques aromatiques. Par acide monocarboxylique aromatique, on entend un composé de formule R'COOH, dans laquelle R' est un radical hydrocarboné aromatique, comprenant 6 à 10 atomes de carbone, et en particulier les radicaux benzoïque et naphtoïque. Ledit radical R' peut en outre être substitué par 1 à 3 radicaux alkyles, saturés ou insaturés, linéaires, ramifiés et/ou cycliques, comprenant 1 à 32 atomes de carbone, notamment 2 à 12, voire 3 à 8 atomes de carbone; et notamment choisis parmi méthyle, éthyle, propyle, isopropyle, butyle, isobutyle, terbutyle, pentyle, isopentyle, néopentyle, cyclopentyle, hexyle, cyclohexyle, heptyle, isoheptyle, octyle ou isooctyle.

Parmi les acides monocarboxyliques non aromatiques susceptibles d'être employés, on peut citer, seul ou en mélange, :
- les acides monocarboxyliques saturés tels que l'acide caproïque, l'acide caprylique, l'acide isoheptanoïque, l'acide 4-éthylpentanoïque, l'acide 2-éthylhexanoïque, l'acide 4,5-diméthylhexanoïque, l'acide 2-heptylheptanoïque, l'acide 3,5,5-triméthylhexanoïque, l'acide octanoïque, l'acide isooctanoïque, l'acide nonanoïque, l'acide décanoïque, l'acide isononanoïque, l'acide laurique, l'acide tridécanoïque, l'acide myristique, l'acide palmitique, l'acide stéarique, l'acide isostéarique, l'acide arachidique, l'acide béhénique, l'acide cérotique (hexacosanoïque); l'acide cyclopentanecarboxylique, l'acide cyclopentaneacétique, l'acide 3-cyclopentylpropionique, l'acide cyclohexanecarboxylique, l'acide cyclohexylacétique, l'acide 4-cyclohexylbutyrique;
- les acides monocarboxyliques insaturés non aromatiques, tels que l'acide caproléique, l'acide obtusilique, l'acide undécylénique, l'acide dodécylénique, l'acide lindérique, l'acide myristoléique, l'acide physétérique, l'acide tsuzuique, l'acide palmitoléique, l'acide oléique, l'acide pétrosélinique, l'acide vaccénique, l'acide élaidique, l'acide gondoïque, l'acide gadoléique, l'acide érucique, l'acide cétoléique, l'acide nervonique, l'acide oléïque, l'acide linoléique, l'acide linolénique, l'acide arachidonique, l'acide ruménique, l'acide éicosapentaènoïque, l'acide docosahexaènoïque;
- les acides conjugués tels que l'acide stillinguique (10:2 2t,4c), l'acide ruménique (18:2, 9c,11t), l'acide linoléïque conjugué (18:2 10t,12c), l'acide linolénique conjugué (18:3 9c,11t,15c), l'acide linolénique conjugué (18:3 6c,9c,11t) et l'acide alpha-éléostéarique (18:3 9c,11t,13t), seuls ou en mélange.

Parmi les acides monocarboxyliques aromatiques susceptibles d'être employés, on peut citer, seul ou en mélange, l'acide benzoïque, l'acide o-toluique, l'acide m-toluique, l'acide p-toluique, l'acide 1-naphtoïque, l'acide 2-naphtoïque, l'acide 4-tert-butyl-benzoïque, l'acide 1-méthyl-2-naphtoïque, l'acide 2-isopropyl-1-naphtoïque.

On utilise un mélange d'acides monocarboxyliques non aromatiques et d'acides monocarboxyliques aromatiques. La proportion de chacun des acides pourra être choisie par l'homme du métier de manière à obtenir un polymère présentant la solubilité désirée. Ainsi, à titre d'indication, si l'on souhaite un polymère plutôt soluble dans les esters courts, de type acétate d'éthyle ou de butyle, l'acide mono-carboxylique non aromatique, ou leur mélange, représente de préférence 1 à 80% en poids, notamment 5 à 50% en poids, voire 8 à 40% en poids, du poids total du mélange d'acides monocarboxyliques, et l'acide monocarboxylique aromatique, ou leur mélange, représente de préférence 20 à 99% en poids, notamment 50 à 95% en poids, mieux encore de 60 à 92% en poids, du poids total dudit mélange. Si l'on souhaite un polymère plutôt soluble dans les huiles cosmétiques (esters gras, alcanes par exemple), de préférence l'acide monocarboxylique non aromatique, ou leur mélange, représente 10 à 99% en poids, notamment 50 à 95% en poids, voire 55 à 90% en poids, du poids total du mélange d'acides monocarboxyliques, et l'acide monocarboxylique aromatique, ou leur mélange, représente de préférence 1 à 90% en poids, notamment 5 à 50% en poids, mieux encore de 10 à 45% en poids, du poids total dudit mélange.

De préférence, on peut utiliser comme acide monocarboxylique, l'acide laurique, l'acide palmitique, l'acide stéarique, l'acide béhénique, l'acide 2-éthylhexanoïque, l'acide isooctanoïque, l'acide isononanoïque, l'acide isostéarique, l'acide benzoïque, l'acide 4-tert-butyl-benzoïque, l'acide o-toluique, l'acide m-toluique, l'acide 1-naphtoïque, l'acide caproléique, l'acide obtusilique, l'acide undécylénique, l'acide dodécylénique, l'acide lindérique, l'acide myristoléique, l'acide physétérique, l'acide tsuzuique, l'acide palmitoléique, l'acide oléique, l'acide linoléique, l'acide linolénique, l'acide arachidonique, et leurs mélanges.

L'acide monocarboxylique, ou leur mélange, représente de préférence 0,1 à 80% en poids, notamment 0,5 à 70% en poids, voire 1 à 65% en poids, et mieux 1,5 à 60% en poids, du poids total du polycondensat formant le squelette polymérique.

Un autre constituant nécessaire pour la préparation des polycondensats selon l'invention est un acide polycarboxylique, et/ou un anhydride cyclique d'un tel acide polycarboxylique ; ainsi que leurs mélanges.

Ledit acide polycarboxylique peut notamment être choisi parmi les acides polycarboxyliques linéaires, ramifiés et/ou cycliques, saturés ou insaturés, voire aromatiques, comprenant 2 à 50, notamment 2 à 40, atomes de carbone, en particulier 3 à 36, voire 3 à 18, et encore mieux 4 à 12 atomes de carbone, voire 5 à 10 atomes de carbone; ledit acide comprenant au moins deux groupes carboxyliques COOH, de préférence de 2 à 4 groupes COOH; et pouvant comprendre 1 à 10 hétéroatomes, de préférence 1 à 6, identiques ou différents, choisis parmi O, N et S; et/ou pouvant comprendre au moins un radical perfluoré choisi parmi CF₂- (divalent) ou -CF₃.

De préférence, ledit acide polycarboxylique est aliphatique saturé, linéaire et comprend 2 à 36 atomes de carbone, notamment 3 à 18 atomes de carbone, voire 4 à 12 atomes de carbone; ou bien est aromatique et comprend 8 à 12 atomes de carbone. Il comprend de préférence 2 à 4 groupes COOH.

Ledit anhydride cyclique d'un tel acide polycarboxylique peut notamment répondre à l'une des formules suivantes : 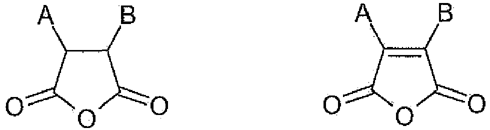 dans lesquelles les groupements A et B sont, indépendamment l'un de l'autre, :
- un atome d'hydrogène,
- un radical carboné, aliphatique, saturé ou insaturé, linéaire, ramifié et/ou cyclique, ou bien aromatique; comprenant 1 à 16 atomes de carbone, notamment 2 à 10 atomes de carbone, voire 4 à 8 atomes de carbone, notamment méthyle ou éthyle;
- ou bien A et B pris ensemble forment un cycle comprenant au total 5 à 14, notamment 5 à 10, voire 6 à 7 atomes de carbone, saturé ou insaturé, voire aromatique.

De préférence, A et B représentent un atome d'hydrogène ou forment ensemble un cycle aromatique comprenant au total 6 à 10 atomes de carbone.

Parmi les acides polycarboxyliques ou leurs anhydrides, susceptibles d'être employés, on peut citer, seul ou en mélange :
- les acides dicarboxyliques tels que l'acide décanedioïque, l'acide dodécanedioïque, l'acide cyclopropanedicarboxylique, l'acide cyclohexanedicarboxylique, l'acide cyclobutanedicarboxylique, l'acide naphtalène-1,4-dicarboxylique, l'acide naphtalène-2,3-dicarboxylique, l'acide naphtalène-2,6-dicarboxylique, l'acide subérique, l'acide oxalique, l'acide malonique, l'acide succinique, l'acide phtalique, l'acide téréphtalique, l'acide isophtalique, l'acide tétrahydrophtalique, l'acide hexahydrophtalique, l'acide pimélique, l'acide sébacique, l'acide azélaïque, l'acide glutarique, l'acide adipique, l'acide fumarique, l'acide maléïque, l'acide itaconique, les dimères d'acides gras (notamment en C36) tels que les produits commercialisés sous les dénominations Pripol 1006, 1009, 1013 et 1017, par Uniqema;
- les acides tricarboxyliques tels que l'acide cyclohexanetricarboxylique, l'acide trimellitique, l'acide 1,2,3-benzènetricarboxylique, l'acide 1,3,5-benzènetricarboxylique;
- les acides tétracarboxyliques tels que l'acide butanetétracarboxylique et l'acide pyroméllitique,
- les anhydrides cycliques de ces acides et notamment l'anhydride phtalique, l'anhydride trimellitique, l'anhydride maléïque et l'anhydride succinique.

De préférence, on peut utiliser l'acide adipique, l'anhydride phtalique et/ou l'acide isophtalique, et encore mieux l'acide isophtalique seul.

On peut également citer les acides polycarboxyliques choisis parmi, seul ou en mélange :
- (i) les acides polycarboxyliques à chaîne saturée ou insaturée, linéaire ou ramifiée, comprenant au moins un hétéroatome choisi parmi O, N et/ou S, notamment 1 à 10 hétéroatomes identiques ou différents, et/ou comprenant au moins un radical perfluoré -CF₂- ou -CF₃ et possédant par ailleurs au moins 2 groupes carboxyliques COOH, notamment 2 à 4 groupes COOH; et/ou un anhydride cyclique d'un tel acide polycarboxylique;
   et/ou
- (ii) les acides polycarboxyliques hétérocycliques, saturé ou insaturé, voire aromatique, comprenant au moins un hétéroatome choisi parmi O, N et/ou S, notamment 1 à 10, voire 1 à 4, hétéroatomes identiques ou différents, et au moins 2 groupes carboxyliques COOH, notamment 2 à 4 groupes COOH; et/ou un anhydride cyclique d'un tel acide polycarboxylique;
   et/ou
- (iii) les acides polycarboxyliques dérivés de sucre, susceptibles d'être obtenus notamment par oxydation d'un aldose, et comprenant au moins 2 groupes carboxyliques COOH, notamment 2 ou 3 groupes COOH; et/ou un anhydride cyclique d'un tel acide polycarboxylique;
   et/ou
- (iv) l'anhydride itaconique et le 1,4-monoanhydride de l'acide 1,4,5,8-naphthalenetetracarboxylique;
   et/ou
- (v) les aminoacides polycarboxyliques (y compris hétérocycliques), c'est-à-dire les acides polycarboxyliques à chaîne saturée ou insaturée, linéaire, ramifiée, et/ou cyclique, comprenant éventuellement au moins un hétéroatome choisi parmi O N et/ou S, notamment 1 à 10 hétéroatomes identiques ou différents, et/ou comprenant éventuellement au moins un radical perfluoré -CF₂- ou -CF₃; et comprenant en outre au moins une fonction amine primaire, secondaire ou tertiaire (notamment NR1 R2 avec R1 et R2, indépendamment l'un de l'autre choisis parmi H et alkyl en C1-C12), notamment 1 à 3 fonctions amines, identiques ou différentes, et possédant par ailleurs au moins 2 groupes carboxyliques COOH, notamment 2 à 4 groupes COOH; et/ou un anhydride cyclique d'un tel acide polycarboxylique.

On peut tout particulièrement citer, seul ou en mélange, les acides dicarboxyliques suivants :
(i)
   - l'acide 2,2'-[1,5-pentanediylbis(thio)]bis-acétique
   - l'acide 6,6'-[(1,2-dioxo-1,2-ethanediyl)diimino]bis-hexanoïque
   - l'acide 2,2'-sulfinylbis-acétique
   - l'acide 4,13-dioxo- 3,5,12,14-Tetraazahexadecanedioïque
   - Le poly(ethylene glycol) disuccinate, notamment de masse 250-600
   - Le poly(ethylene glycol)bis(carboxymethyl)éther, notamment de masse 250-600
   - Le poly[oxy(1,2-dicarboxy-1,2-ethanediyl)], notamment de DP < 10
   - L'acide 8-[(carboxymethyl)amino]-8-oxo-octanoïque
   - L'acide 2,2'-[methylenebis(sulfonyl)]bis-acétique
   - L'acide 4,4'-(1,6-hexanediyldiimino)bis[4-oxo-butanoïque]
   - L'acide 4,9-dioxo- 3,5,8,10-tétraazadodecanedioïque
   - L'acide 4-[(1-carboxyethyl)amino]-4-oxo-butanoïque
   - L'acide 6-[(3-carboxy-1-oxopropyl}amino]-hexanoïque
   - La N,N'-(1,6-dioxo-1,6-hexanediyl)bis-glycine
   - La N,N'-(1,6-dioxo-1,6-hexanediyl)bis-phenylalanine
   - La N,N'-(1,3-dioxo-1,3-propanediyl)bis-glycine
   - L'acide 4,4'-(1,4-dioxo-1,4-butanediyl)diimino]bis-butanoïque
   - L'acide 4,4'-[(1,6-dioxo-1,6-hexanediyl)diimino]bis-butanoïque
   - L'acide 6,6'-[1,6-hexanediylbis(iminocarbonylimino)]bis-hexanoïque
   - La N-benzoyl-S-(carboxymethyl)- Cysteine
   - La N,N'-(2,2,3,3-tetrafluoro-1,4-dioxo-1,4-butanediyl)bis- Glycine
   - La N,N'-(2,2,3,3-tetrafluoro-1,4-dioxo-1,4-butanediyl)bis-Alanine
   - L'acide 4,4'-[(2,2,3,3-tetrafluoro-1,4-dioxo-1,4- butanoïque
   - La N,N'-(1,5-dioxo-1,5-pentanediyl)bis- Glycine
   - La N,N'-(1,9-dioxo-1,9-nonanediyl)bis- Glycine
   - La N,N'-(1,10-dioxo-1,10-decanediyl)bis[N-methyl- Glycine
   - Le bis(3-carboxypropyl) ester de l'acide propanedioïque
   - L'acide 7,16-dioxo- 6,8,15,17-Tetraazadocosanedioïque
   - La N-benzoyl-N-(2-carboxyethyl)-Glycine
   - L'acide [2-[(2-carboxymethyl)amino]-2-oxoethyl]- Benzenepropanoïque
   - L'acide [2-[(2-carboxyethyl)amino]-2-oxoethyl]- Benzenepropanoïque
(ii)
   - L'acide 4,7,9,12-Tetraoxapentadecanedioïque
   - L'acide 2,3-Pyridinedicarboxylique
   - L'acide 4-Pyranone-2,6-dicarboxylique
   - L'acide 2,5-Pyrazinedicarboxylique
   - L'acide 2,5-Pyridinedicarboxylique
   - L'acide 2,3-Benzofurandicarboxylique
   - L'acide 7-oxabicyclo[2.2.1]heptane-2,3-dicarboxylique
   - L'acide 3,4-Pyridinedicarboxylique
   - L'acide 2,4-Pyridinedicarboxylique
   - L'acide 3,5-Pyridinedicarboxylique
   - L'acide 2,6-Pyridinedicarboxylique
   - L'acide 1H-Imidazole-4,5-dicarboxylique
   - L'acide 2,3-Quinolinedicarboxylique
   - L'acide 6,6,7,7-tetrafluoro-3-Oxabicyclo[3.2.0]heptane-2,4-dicarboxylique
   - L'acide 2,6-Pyrazinedicarboxylique
   - L'acide 2,6-Dimethyl-3,5-pyridinedicarboxylique
   - L'acide 1-phenyl-1H-Pyrazole-3,4-dicarboxylique
   - L'acide 2,5-Furanedicarboxylique
   - L'acide 3,4-Furanedicarboxylique
   - L'acide 1,2,5-Thiadiazole-3,4-dicarboxylique
   - L'acide 1,4-Dihydro-1,2,4,5-tetrazine-3,6-dicarboxylique
   - L'acide 2,3-Furanedicarboxylique
   - L'acide 3,4-Thiophenedicarboxylique
   - L'acide 1H-1,2,3-Triazole-4,5-dicarboxylique
   - L'acide 2-Methylimidazole-4,5-dicarboxylique
   - L'acide 2,4-Quinolinedicarboxylique
   - L'acide Naphtho[2,1-b]furane-1,2-dicarboxylique
   - L'acide 3,4-Quinolinedicarboxylique
   - L'acide 7-Oxabicyclo[2.2.1]hept-5-ene-2,3-dicarboxylique
   - L'acide 2,3-Quinoxalinedicarboxylique
   - L'acide 1,4-Piperazinedicarboxylique
   - L'acide 2,5-dimethyl- 3,4-Furandicarboxylique
   - L'acide tetrahydro-2,5-Thiophenedicarboxylique
   - L'acide 4-phenyl- 3,5-Pyridinedicarboxylique
   - L'acide Thieno[3,2-b]thiophene-2,5-dicarboxylique
   - L'acide 3-methyl- 2,4-Thiophenedicarboxylique
   - L'acide Naphthostyril-5,6-dicarboxylique
   - L'acide 3-phenyl- 2,4-Quinolinedicarboxylique
   - Le 3,4-Dimethyl-2,5-dicarboxythiophene
   - L'acide 3,4-Diphenyl-2,5-thiophenedicarboxylique
   - L'acide 2,5-diphenyl- 3,4-Furanedicarboxylique
   - L'acide 7-oxo-7H-Benzimidazo[2,1-a]benz[de]isoquinoline-3,4-dicarboxylique
   - L'acide 2,3-dihydro-1,3-dioxo-1H-Benz[de]isoquinoline-6,7-dicarboxylique
   - L'acide 3,4-bis(phenylmethoxy)-2,5-Furandicarboxylique
   - L'acide 4,4'-Bibenzoïque-2,2'-sulfone
   - L'acide 2,1-Diphenyl-m-anthrazoline-4,5-dicarboxylique
   - L'acide 2,4-Pyrimidinedicarboxylique
   - L'acide 2-phenyl- 4,5-Thiazoledicarboxylique
   - L'acide 6-phenyl- 2,3-Pyridinedicarboxylique
   - L'acide 5,6-Dimethyl-2,3-pyrazinedicarboxylique
   - L'acide 3,7-Dibenzothiophenedicarboxylique
   - L'acide 9-oxo-9H-Xanthene-1,7-dicarboxylique
   - L'acide 2-(1,1-dimethylethyl)-H-Imidazole-4,5-dicarboxylique
   - L'acide 6,7-Quinolinedicarboxylique
   - L'acide 6-Methyl-2,3-pyridinedicarboxylique
   - L'acide 4,5-Pyrimidinedicarboxylique
   - L'acide 2-methyl-3,4-Furanedicarboxylique
   - L'acide 1,2-Indolizinedicarboxylique
   - L'acide 2,8-Dibenzothiophenedicarboxylique
   - L'acide 3,6-Pyridazinedicarboxylique
   - L'acide 1,10-Phenanthroline-2,9-dicarboxylique
   - L'acide 1,4,5,6-tetrahydro-5,6-dioxo-2,3-Pyrazinedicarboxylique
   - L'acide 3,4-Dimethoxy-2,5-furandicarboxylique
   - L'acide 2-Ethyl-4,5-imidazoledicarboxylique
   - L'acide 2-Propyl-1H-imidazole-4,5-dicarboxylique
   - L'acide 4-phenyl- 2,5-Pyridinedicarboxylique
   - L'acide 4,5-Pyridazinedicarboxylique
   - L'acide 1,4,5,8-tetrahydro-1,4:5,8-Diepoxynaphthalene-4a,8a-dicarboxylique
   - L'acide 5,5-dioxide-2,8-Dibenzothiophenedicarboxylique
   - L'acide Pyrazolo[1,5-a]pyridine-2,3-dicarboxylique
   - L'acide 2,3-dihydro-1H-Pyrrolizine-1,7-dicarboxylique
   - L'acide 6-methyl-2,4,5-Pyridinetricarboxylique
   - L'acide Pyrrolo[2,1,5-cd]indolizine-5,6,-dicarboxylique
   - L'acide 3,4-bis(2,2,3,3,4,4,4-heptafluorobutyl)-1H-Pyrrole-2,5-dicarboxylique
   - L'acide 6,7,9,10,17,18,20,21-octahydrodibenzo[b,k][1,4,7,10,13,16]hexaoxacyclo-octadecin-2,14-dicarboxylique
   - L'acide 6,7,9,10,17,18,20,21-octahydro-dibenzo[b,k][1,4,7,10,13,16]hexaoxacyclo octadecin-2,13-dicarboxylique
   - L'acide 2-methyl- 3,4-Quinolinedicarboxylique
   - L'acide 4,7-Quinolinedicarboxylique
   - L'acide 3,5-Isoxazoledicarboxylique
   - L'acide 2-(trifluoromethyl)- 3,4-Furanedicarboxylique
   - L'acide 5-(trifluoromethyl)- 2,4-Furanedicarboxylique
   - L'acide 6-methyl-2,4-quinolinedicarboxylique
   - L'acide 5-oxo-1,2-Pyrrolidinedicarboxylique
   - L'acide 5-Ethyl-2,3-pyridinedicarboxylique
   - L'acide 1,2-dihydro-2-oxo- 3,4-quinolinedicarboxylique
   - L'acide 4,6-Phenoxathiindicarboxylique
   - L'acide 10,10-dioxide 1,9-phenoxathiindicarboxylique
   - L'acide 3,4-dihydro-2H-1,4-Thiazine-3,5-dicarboxylique
   - L'acide 2,7-Di(tert-butyl)-9,9-dimethyl-4,5-xanthenedicarboxylique
   - L'acide 6-methyl-2,3-Quinoxalinedicarboxylique
   - L'acide 3,7-Quinolinedicarboxylique
   - L'acide 2,5-Quinolinedicarboxyliquer
   - L'acide 2-Methyl-6-phenyl-3,4-pyridinedicarboxylique
   - L'acide 3,4-dimethyl- thieno[2,3-b]thiophene-2,5-dicarboxylique
   - L'acide 3,4-Dimethoxythiophene-2,5-dicarboxylique
   - L'acide 5-methyl-3,4-Isoxazoledicarboxylique
   - L'acide 2,6-bis(aminocarbonyl)-3,5-Pyridinedicarboxylique
   - L'acide 3,5-bis(aminocarbonyl)-2,6-Pyrazinedicarboxylique
   - L'acide 2,3-Pyridinedicarboxylique
   - L'acide 6-(1,1-dimethylethyl)-2-ethyl-3,4-Pyridinedicarboxylique
   - L'acide 3-methyl-5-phenyl-2,4-Thiophenedicarboxylique
   - L'acide 1,2-dihydro-2-oxo-6-phenyl-3,5-Pyridinedicarboxylique
   - L'acide 8-methyl-2,4-Quinolinedicarboxylique
   - L'acide 4-ethyl-2,6-dimethyl-3,5-Pyridinedicarboxylique
   - L'acide 5-(phenoxymethyl)-2,4-Furanedicarboxylique
   - L'acide 5-(acetylamino)-3-methyl-2,4-Thiophenedicarboxylique
   - L'acide 2-(4-heptylphenyl)- 4,8-Quinolinedicarboxylique
   - L'acide 2,8-bis(4-heptylphenyl)-pyrido[3,2-g]quinoline-4,6-dicarboxylique
   - L'acide 1,2,3,4,6,7,8,9-octahydro-2,8-dioxo-pyrido[3,2]quinoline-3,7-dicarboxylique
   - L'acide 2,8-dimethyl- Pyrido[3,2-g]quinoline-3,7-dicarboxylique
   - L'acide 5,6-Quinolinedicarboxylique
   - L'acide 6-ethyl-2-methyl- Cinchomeronique
   - L'acide 2-methyl-6-propyl-Cinchomeronique
   - L'acide 6-isopropyl-2-methyl- Cinchomeronique
   - L'acide 6-tert-butyl-2-methyl- Cinchomeronique
   - L'acide 1,4-dimethyl-7-Oxabicyclo[2.2.1]heptane-2,3-dicarboxylique
   - L'acide 1,2-dihydro-2-oxo-3,8-Quinolinedicarboxylique
   - L'acide 1,2-dihydro-2-oxo-3,6-Quinolinedicarboxylique
   - L'acide 1,2-dihydro-2-oxo-3,7-Quinolinedicarboxylique
   - L'acide 3,7-dimethyl-2,8-diphenyl-Pyrido[3,2-g]quinoline-4,6-dicarboxylique
   - L'acide 8-methyl-2,3-Quinolinedicarboxylique
   - L'acide 3-[[(1,1-dimethylethyl)amino]sulfonyl]-2,5-Thiophene-dicarboxylique
   - L'acide 4-(acetylamino)-2,3-Thiophenedicarboxylique
   - L'acide 2,5-Pyridinedicarboxylique
   - L'acide 2,6-Pyridinedicarboxylique
   - L'acide 2,4-Thiophenedicarboxylique
   - L'acide 2,5-Thiophenedicarboxylique
   - L'acide 1,4-pyrane-2,6-dicarboxylique
(iii)
   - L'acide Ribarique
   - L'acide Glucarique
   - L'acide Xylarique
   - L'acide Arabinarique
   - L'acide Mannarique
   - L'acide Idarique
   - L'acide Altrarique
   - L'acide L-Glucarique
   - L'acide L-Arabinarique
   - L'acide Allarique
   - L'acide Galactarique
   - L'acide meso-Tartarique
   - L'acide D-Glucarique
   - L'acide L-Idarique
   - L'acide Hexarique
   - L'acide 2,3-dihydroxy- butanedioique
   - L'acide D-Tartarique
   - L'acide DL-Tartarique
   - L'acide D-Glucarique
   - l'acide tartarique
   - L'acide Tetrahydroxysuccinique
   - L'acide 2-carboxy-2,3-dideoxy D-manno-2-Octulopyranosonique
   - L'acide methyl 3-deoxy-D-arabino-2-Heptulopyranosarique
   - L'acide D-lyxo-2-Heptulopyranosarique
   - L'acide 2,6-anhydro-L-glycero-L-galacto-Heptarique
(iv)
   - le 1,4-monoanhydride de l'acide 1,4,5,8-Naphthalenetetracarboxylique
   - l'anhydride itaconique
(v)
   - l'acide 1,4-dihydro-4-oxo-2,6-Pyridinedicarboxylique
   - l'acide 2,6-Piperidinedicarboxylique
   - l'acide 1H-Pyrrole-3,4-dicarboxylique
   - l'acide 4-amino-2,6-dicarboxylique
   - l'acide 1-methyl-1H-Pyrazole-3,4-dicarboxylique
   - l'acide 2,3-Piperidinedicarboxylique
   - l'acide 1-methyl-1H-Imidazole-4,5-dicarboxylique
   - l'acide 2,4-Thiazolidinedicarboxylique
   - l'acide 1-(phenylmethyl)-1H-Imidazole-4,5-dicarboxylique
   - l'acide 5-amino-6-oxo-2,3-Piperidinedicarboxylique
   - l'acide 5-amino-6-oxo-2,4-Piperidinedicarboxylique
   - l'acide 5-amino-6-oxo-2,3-Piperidinedicarboxylique
   - l'acide 5-amino-6-oxo-, [2S-(2α,4β,5α)]- 2,4-Piperidinedicarboxylique
   - l'acide (2S,4R)-2,4-Pyrrolidinedicarboxylique
   - l'acide-(2S-cis)-2,4-Pyrrolidinedicarboxylique
   - l'acide 2-amine-1H-Imidazole-4,5-dicarboxylique
   - l'acide 2,5-Pyrrolidinedicarboxylique
   - l'acide 4-amino-3,5-Isothiazoledicarboxylique
   - l'acide 1-methyl- 1H-Pyrazole-3,5-dicarboxylique
   - l'acide 7-(diethylamino)-2-oxo-2H-1-Benzopyran-3,4-dicarboxylique
   - l'acide 3,4-diethyl-1H-Pyrrole-2,5-dicarboxylique
   - l'acide 1-phenyl-1H-Pyrrole-3,4-dicarboxylique
   - l'acide cis-2,3-Piperazinedicarboxylique
   - l'acide 2,3-Piperazinedicarboxylique
   - l'acide 2,5-Piperazinedicarboxylique
   - l'acide 2,6-Piperazinedicarboxylique
   - l'acide 2-amino-3,5-Pyridinedicarboxylique
   - l'acide 2-methyl-Pyrrole-3,4-dicarboxylique
   - l'acide 4-(methylamino)-2,6-Pyridinedicarboxylique
   - l'acide 2-amino-6-methyl-3,4-Pyridinedicarboxylique
   - l'acide 5-amino-2-methyl-3,4-Pyridinedicarboxylique
   - l'acide 2-amino-6-methyl- 3,5-Pyridinedicarboxylique
   - l'acide 2,5-dimethyl-Pyrrole-3,4-dicarboxylique
   - l'acide 2,5-dimethyl-Pyrrole-3,4-dicarboxylique
   - l'acide 2-amino-6-hydroxy- 3,5-Pyridinedicarboxylique
   - l'acide 2,4-Pyrrolidinedicarboxylique
   - l'acide 1H-Indole-2,4-dicarboxylique
   - l'acide 1H-Indole-2,6-dicarboxylique
   - l'acide 1H-indole-2,5-dicarboxylique
   - l'acide 5-phenyl-2,4-Pyrrolidinedicarboxylique
   - l'acide 5-methyl-2,4-Pyrrolidinedicarboxylique
   - l'acide trans-2,4-Azetidinedicarboxylique
   - l'acide cis-2,4-Azetidinedicarboxylique
   - l'acide 3,5-Piperidinedicarboxylique
   - l'acide 2,3-Pyrrolidinedicarboxylique
   - l'acide 2,3-Azetidinedicarboxylique
   - l'acide 3,4-Pyrrolidinedicarboxylique
   - l'acide 2,3-dihydro-6H-1,4-Dioxino[2,3-c]pyrrole-5,7-dicarboxylique
   - l'acide 1H-Imidazole-2,4-dicarboxylique
   - l'acide 1-butyl-1H-Pyrrole-2,3-dicarboxylique
   - l'acide 3-amino-1-oxide-2,4-Pyridinedicarboxylique
   - l'acide 2,3-dihydro-5-phenyl-1H-Pyrrolizine-6,7-dicarboxylique
   - l'acide 3a,4,5,9b-tetrahydro-3H-Cyclopenta[c]quinoline-4,6-dicarboxylique
   - l'acide 3a,4,5,9b-tetrahydro-3H-Cyclopenta[c]quinoline-4,8-dicarboxylique
   - l'acide 2,3-dihydro-1H-Imidazole-4,5-dicarboxylique
   - l'acide 5-amino-6-methyl-Lutidinique
   - l'acide 1H-Indole-3,7-dicarboxylique
   - l'acide 3,3-dimethyl-2,6-Piperidinedicarboxylique
   - l'acide 1-butyl-2,5-Pyrrolidinedicarboxylique
   - l'acide 1H-Indole-4,6-dicarboxylique
   - l'acide 1-(phenylmethyl)-3,4-Pyrrolidinedicarboxylique
   - l'acide 3-(carboxymethyl)-1H-lndole-2,6-dicarboxylique
   - l'acide 3,4-bis(2,2,2-trifluoroethyl)-1H-Pyrrole-2,5-dicarboxylique
   - l'acide 9-hexyl-9H-Carbazole-3,6-dicarboxylique
   - l'acide 3-methyl-5-(1-piperazinylsulfonyl)-2,4-Thiophenedicarboxylique
   - l'acide 2,3,4,9-tetrahydro-1H-Carbazole-5,7-dicarboxylique
   - l'acide 2,3-dimethyl-1H-Indole-4,6-dicarboxylique
   - l'acide 7-amino-1,4-dihydro-4-oxo-3,6-Quinolinedicarboxylique
   - l'acide 5-amino-3-methyl-2,4-Thiophenedicarboxylique
   - l'acide (m-tolylimino)di-Acetique
   - l'acide (o-tolylimino)di-Acetique
   - la D-Cystathionine
   - l'acide Phenethyliminodiacetique
   - l'acide 2-Benzyl-2,2'-iminodiacetique
   - la L-α-glutamyl-L-alanyl-L-Alanine
   - l'acide N,N'-Dibenzylethylenediaminediacetique
   - la N-L-γ-glutamyl-D-Alanine
   - la Glycyl-L-glutamyl glycine
   - la N-(carboxymethyl)-N-(tetrahydro-1,1-dioxido-3-thienyl)-Glycine
   - la N-(2-carboxyethyl)-N-phenyl-beta-Alanine
   - la N-(carboxymethyl)-N-octyl-glycine
   - l'acide N-(tert-Butoxycarbonyl)iminodiacetique
   - la N-(carboxymethyl)-L-Alanine
   - la N-(6-aminohexyl)-N-(carboxymethyl)-Glycine
   - la N-(carboxymethyl)-N-tetradecyl-Glycine
   - la N-(1-carboxyethyl)-D-Alanine
   - la N-(carboxymethyl)-D-Alanine
   - l'acide decyliminodiacetique
   - l'acide 3,3'-(dimethylhydrazono)bis-propanoique
   - la N-(carboxymethyl)-N-[2-(2,6-dioxo-4-morpholinyl)ethyl]-glycine
   - la N-alpha-aspartyl-glycine
   - la N-beta-aspartyl-glycine
   - la N-L-alpha-aspartyl-beta-Alanine
   - l'acide 3,4-Xylylamino-N,N-diacetique.
   - la N-(1-carboxyethyl)-Alanine
   - la N-(carboxymethyl)-Alanine
   - la N,N'-methylenebis-glycine
   - la N-(aminomethyl)-N-(carboxymethyl)-glycine
   - la N-(aminomethyl)-N-(carboxymethyl)-glycine
   - l'acide 2,2'-(methylhydrazono)bis-acetique
   - la N-(2-carboxyethyl)-N-(4-methylphenyl)-beta-Alanine
   - la N-(2-carboxyethyl)-N-(3-methylphenyl)-beta-Alanine
   - la 3-[(carboxymethyl)amino]-Alanine
   - la D-alpha-aspartyl-D-Alanine
   - la N-(2-carboxyethyl)-N-(1-oxohexadecyl)-beta-Alanine
   - la N-(2-carboxyethyl)-N-(1-oxodecyl)-beta-Alanine
   - la N-(2-carboxyethyl)-N-(1-oxotetradecyl)-beta-Alanine
   - L'acide amino[(carboxymethyl)thio]- Acetique
   - la N,N'-1,6-hexanediylbis-beta-Alanine
   - la N-(carboxymethyl)-N-phenyl-beta-Alanine
   - la N-(3-carboxyethyl)-L-Alanine
   - l'acide L-glutamique
   - l'acide L-aspartique.

On peut également citer, seul ou en mélange, les acides tricarboxyliques et tétracarboxyliques suivants, ainsi que leurs anhydrides:
- L'acide 3,3',3"-[1,2,3-propanetriyltris(oxy)]tris-propanoïque
- L'acide Pyrazinetricarboxylique
- L'acide 4-(3-carboxyphenyl)-2,5-Pyridinedicarboxylique
- L'acide 3-(carboxymethyl)-2,4-Quinolinedicarboxylique
- L'acide 3-(carboxymethyl)-1H-Indole-2,5-dicarboxylique
- L'acide 3-C-carboxy-2-deoxy-D-threo-Pentarique
- L'acide Hydroxycitrique
- L'acide D-glucopyranuronosyl-D-arabino-2-Hexulofuranosidarique
- L'acide 2,3,5,6-Pyridinetetracarboxylique
- la N,N'-1,2-ethanediylbis[N-(carboxymethyl)-β-alanine
- l'acide L-α-aspartyl-L-Aspartique
- l'acide 4-[bis(carboxymethyl)amino]-Benzoique
- l'acide 7-[bis(carboxymethyl)amino]-Heptanoique
- l'acide N-(2-carboxyethyl)-Aspartique
- l'acide 3-[bis(2-carboxyethyl)amino]-Benzoique
- l'acide 4-[bis(2-carboxyethyl)amino]-Benzoique.

De préférence, on peut utiliser l'acide 6,6'-[(1,2-dioxo-1,2-ethanediyl)diimino]bis-hexanoïque, l'acide 2,2'-sulfinylbis-acétique, l'acide 4,13-dioxo-3,5,12,14-tetraazahexadecanedioïque, le poly(ethylene glycol)disuccinate, le poly(ethylene glycol)bis(carboxymethyl)éther, l'acide 8-[(carboxymethyl)amino]-8-oxo-octanoïque, l'acide 2,2'-[methylenebis(sulfonyl)]bis-acétique, l'acide 4,4'-(1,6-hexanediyldiimino)bis[4-oxo-butanoïque], l'acide 4,9-dioxo-3,5,8,10-tétraazadodecanedioïque, l'acide 4-[(1-carboxyethyl)amino]-oxo-butanoïque, l'acide 6-[(3-carboxy-1-oxopropyl)amino]-hexanoïque, la N,N'-(1,6-dioxo-1,6-hexanediyl)bis-glycine, la N,N'-(1,3-dioxo-1,3-propanediyl)bis-glycine, l'acide 4,7,9,12-tétraoxapentadecanedioïque, l'acide 4-Pyranone-2,6-dicarboxylique, l'acide 2,5-Pyrazinedicarboxylique, l'acide 1H-Imidazole-4,5-dicarboxylique, l'acide 2,6-Pyrazinedicarboxylique, l'acide 2,5-Furanedicarboxylique, l'acide 3,4-Furanedicarboxylique, l'acide 2,3-Furanedicarboxylique, l'acide 2,5-diphenyl-3,4-Furanedicarboxylique, l'acide 2-methyl-3,4-Furanedicarboxylique, l'acide D-Tartarique, l'acide DL-Tartarique, l'acide L-Tartarique, l'acide Galactarique, l'acide D-Glucarique; L'acide 2,5-Pyridinedicarboxylique, l'acide 2,5-Pyrrolidinedicarboxylique, l'acide 1-phenyl-1H-Pyrrole-3,4-dicarboxylique, l'acide 2,4-Pyrrolidinedicarboxylique, l'acide 5-phenyl-2,4-Pyrrolidinedicarboxylique, l'acide 3,5-Piperidinedicarboxylique, l'acide 3,4-Pyrrolidinedicarboxylique, l'acide 1-butyl-2,5-Pyrrolidinedicarboxylique, l'acide 1-(phenylmethyl)-3,4-Pyrrolidinedicarboxylique, la N-(2-carboxyethyl)-N-phenyl-β-Alanine, la N-(carboxymethyl)-N-octyl-glycine, la N-(1-carboxyethyl)-L-Alanine, l'acide L-glutamique, l'acide L-aspartique, l'acide N-(2-carboxyethyl)-Aspartique; et leurs mélanges.

On préfèrera tout particulièrement l'acide 2,2'-sulfinylbis-acétique, l'acide 2,2'-[methylenebis(sulfonyl)]bis-acétique, la N,N'-(1,3-dioxo-1,3-propanediyl)bis-glycine, l'acide 2,5-Furanedicarboxylique, l'acide D-Tartarique, l'acide DL-Tartarique, l'acide L-Tartarique, l'acide Galactarique, l'acide L-glutamique, l'acide L-aspartique, et leurs mélanges.

Ledit acide polycarboxylique et/ou son anhydride cyclique, et leurs mélanges, représente de préférence 5 à 60% en poids, notamment 10 à 40% en poids, et mieux 12 à 25% en poids, du poids total du polycondensat formant le squelette polymérique.

Le polycondensat susceptible de conduire au squelette polymérique doit par ailleurs comporter au moins un groupe OH libre, susceptible de réagir chimiquement avec les groupes isocyanates portés par le groupe de jonction; ceci implique notamment qu'il possède un indice d'hydroxyle non nul, notamment compris entre 5 et 200, et mieux compris entre 10 et 150, exprimé en mg d'hydroxyde de potassium par g de polycondensat.

Ledit squelette polymérique porteur de groupes hydroxyle OH peut donc être schématisé de la manière suivante: POL-(OH)n, avec n = entier supérieur ou égal à 1.

Le polymère selon l'invention comporte également au moins un groupe de jonction lié audit squelette polymérique et capable d'établir des liaisons H avec un ou plusieurs groupes de jonction partenaires, de nature chimique identique ou différente, chaque appariement d'un groupe de jonction faisant intervenir au moins 3 liaisons H (hydrogène), de préférence au moins 4 liaisons H, préférentiellement 4 liaisons H.

Par "groupe de jonction", on entend au sens de l'invention, tout groupe fonctionnel comportant des groupes donneurs ou accepteurs de liaisons H, et capable d'établir au moins trois liaisons H, de préférence au moins 4 liaisons H, préférentiellement 4 liaisons H, avec un groupe de jonction partenaire, identique ou non. Par "groupe de jonction partenaire", on entend au sens de l'invention, tout groupe de jonction pouvant établir des liaisons H avec un ou plusieurs groupes de jonction d'un même ou d'un autre polymère selon l'invention. Les groupes de jonction peuvent être de nature chimique identique ou différente. S'ils sont identiques, ils peuvent alors établir des liaisons H entre eux et sont alors appelés groupes de jonction auto-complémentaires. S'ils sont différents, ils sont choisis de telle façon qu'ils soient complémentaires vis à vis des interactions H.

Ledit groupe de jonction porte au moins un groupe isocyanate, susceptible de réagir avec les groupements hydroxyles portés par le squelette polymérique, de manière à former une fonction uréthanne, liant ledit squelette audit groupe de jonction. Il est à noter que ladite fonction uréthanne est à considérer comme faisant partie intégrante du groupe de jonction; elle est donc susceptible d'intervenir dans la formation de 3 liaisons H par ledit groupe de jonction.

Ledit groupe de jonction porteur de groupes isocyanates peut être schématisé de la manière suivante: OCN-A-NCO ou B-NCO, selon qu'il comporte un ou deux groupes NCO.

Pour le radical divalent A, le radical R2 peut notamment être H, ou bien :
- un groupe alkyle en C₃-C₃₂ ;
- un groupe cycloalkyle en C₄-C₁₂ ;
- un groupe aryle en C₄-C₁₂ ;
- un groupe aryl(C₄-C₁₂) alkyle en C₁-C₁₈
- un groupe alcoxy en C₁-C₄ ;
- un groupe arylalcoxy, en particulier un groupe aryle (C₁-C₄) alcoxy ;
- un hétérocycle en C₄-C₁₂
ou une combinaison de ces radicaux, qui peuvent éventuellement être substitués par une fonction amino, ester et/ou hydroxy.

De préférence R² représente H, CH₃, C₁₃H₂₇, C₇H₁₅, phényle, isopropyle, isobutyle, n-butyle, tert-butyle ou propyle.

De manière plus préférée, le radical divalent A peut être de formule : 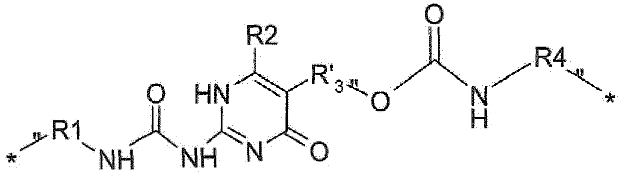 dans laquelle R1 et R2 sont tels que ci-dessus, et R'3 et R4, identiques ou différents, représentent un radical carboné divalent choisi parmi un groupe alkyle linéaire ou ramifié en C₁-C₃₀ ou un groupe cycloalkyle en C₄-C₁₂ ou un groupe aryle en C₄-C₁₂; ou leur mélanges.

Encore plus préférentiellement, le radical A peut être de formule: 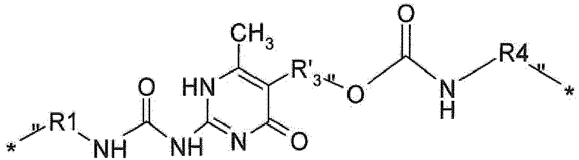 dans laquelle R1, R'3 et R4 sont tels que ci-dessus définis.

Préférentiellement, les radicaux R1, R3, R'3 et R4, indépendamment l'un de l'autre, peuvent être choisis parmi les radicaux suivants : méthylène, 1,2-éthylène, 1,6-héxylène, 1,4-butylène, 1,6-(2,4,4-triméthylhexylène), 1,4-(4-méthylpentylène), 1,5-(5-méthylhexylène); 1,6-(6-méthylheptylène); 1,5-(2,2,5-triméthylhexylène), 1,7-(3,7-diméthyloctylène); 4,4'-méthylènebiscyclohexylène; 2-méthyl-1,3-phénylène; 4-méthyl-1,3-phénylène ; 4,4'-biphénylèneméthylène, 1,2-tolylène, 1,4-tolylène, 2,4-tolylène, 2,6-tolylène; 1,5-naphtylène; 4,4'-methylenebis(phényl); tétraméthyl xylylène; le radical divalent dérivé de l'isophorone.

De façon particulièrement préférée, R1, R3, R'3 et R4 représentent, indépendamment l'un de l'autre, -(CH₂)₂-, -(CH₂)₆-, -CH₂CH(CH₃)CH₂C(CH₃)₂CH₂CH₂-, ou un radical -isophorone-; et mieux R1=R4=-isophorone- et R'3=-(CH₂)₂-, ce qui conduit au radical très préféré suivant :

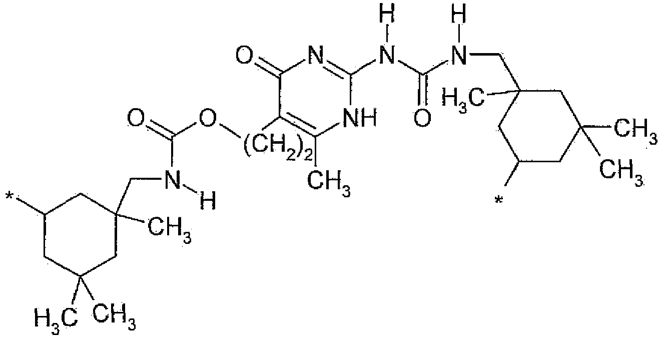

Ainsi, les groupes de jonction OCN-A-NCO particulièrement préférés peuvent également être choisis parmi les composés de formule: 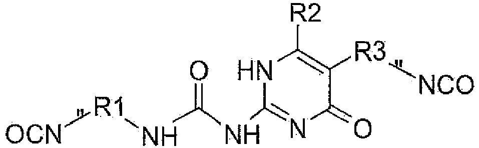 dans laquelle R1, R2 et R3 sont tels que définis ci-dessus, et de manière préférée parmi les composés de formule : 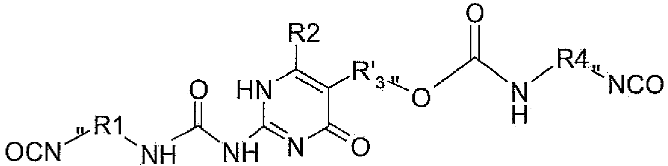 dans laquelle R'3 et R4, identiques ou différents, représentent un radical carboné divalent choisi parmi un groupe alkyle linéaire ou ramifié en C₁-C₃₀ ou un groupe cycloalkyle en C₄-C₁₂ ou un groupe aryle en C₄-C₁₂; ou leur mélanges.

Encore plus préférentiellement, le groupe de jonction peut être de formule :

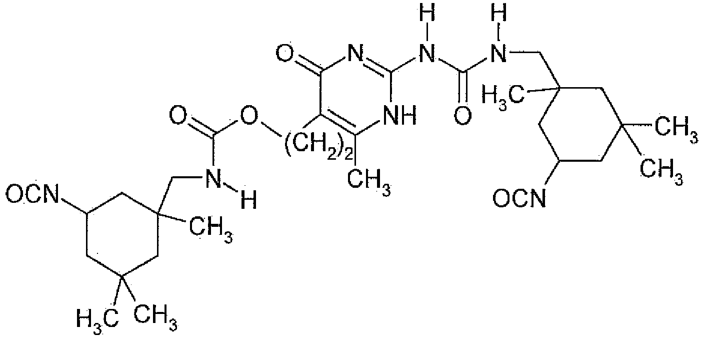

Pour le radical monovalent B , de préférence R1 représente un groupe alkyle en C2-C10, et peut être choisi parmi 1,2-éthylène, 1,6-héxylène, 1,4-butylène, 1,6-(2,4,4-triméthylhexylène), 1,4-(4-méthylpentylène), 1,5-(5-méthylhexylène), 1,6-(6-méthylheptylène), 1,5-(2,2,5-triméthylhexylène), 1,7-(3,7-diméthyloctylène);-isophorone-, 4,4'-méthylènebiscyclohexylène, tolylène, 2-méthyl-1,3-phénylène, 4-méthyl-1,3-phénylène, 4,4-bisphénylèneméthylène.

Préférentiellement, R1 représente -isophorone-, -(CH₂)₂-, -(CH₂)₆-, -CH₂CH(CH₃)-CH₂-C(CH₃)₂-CH₂-CH₂, 4,4'-méthylènebiscyclohexylène, 2-méthyl-1,3-phénylène. De préférence, R2 représente un radical alkyle en C1-C16, voire en C1-C10 et notamment méthyl, éthyle, isopropyl, propyl, isobutyl, n-butyl, tert-butyle, ou - CH(C₂H₅)(C₄H₉).

De préférence, R3 représente H.

Préférentiellement, on peut avoir :
- R₁=-isophorone-, R2=méthyl et R3=H, ce qui conduit au radical : 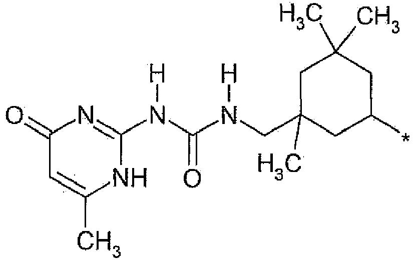
- R₁=-(CH₂)₆-, R2=méthyl et R3=H, ce qui conduit au radical : 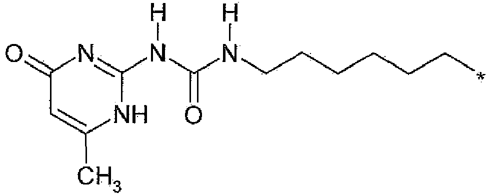
- R₁=-(CH₂)₆-, R2=isopropyl et R3=H, ce qui conduit au radical :

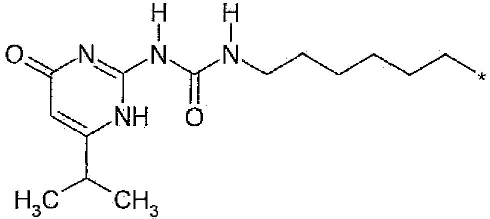

Le groupe de jonction B-NCO peut donc être choisi parmi les composés de formule : 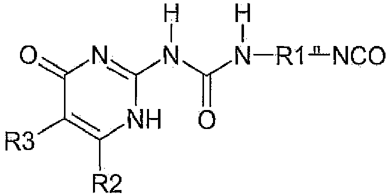 dans laquelle R1, R2 et R3 sont tels que définis ci-dessus;
et peut notamment être choisi parmi les groupes suivants :

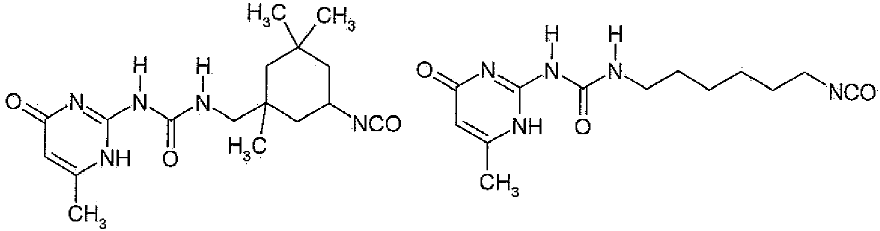

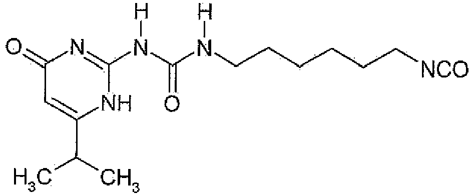

De préférence, le polymère selon l'invention présente une viscosité, mesurée à 125°C, comprise entre 30 et 5000 mPa.s, notamment entre 150 et 3500 mPa.s, voire entre 500 et 3000 mPa.s et encore mieux entre 750 et 2500 mPa.s. Cette viscosité est mesurée de la manière décrite avant les exemples.

Par ailleurs, le polymère selon l'invention est avantageusement soluble dans les milieux huileux cosmétiques usuellement employés, et notamment dans les huiles végétales, les alcanes, les esters gras, les esters courts, les alcools gras, les huiles de silicone, et plus particulièrement dans les milieux comprenant l'isododécane, le Parléam, l'isononanoate d'isononyle, l'octyldodécanol, la phényl triméthicone, le benzoate d'alkyle en C12-C15, l'acétate de butyle, l'acétate d'éthyle et/ou la D5 (décaméthylcyclopentasiloxane).

Par soluble, on entend que le polymère forme une solution limpide dans au moins un solvant choisi parmi l'isododécane, le Parléam, l'isononanoate d'isononyle, l'octyldodécanol, la phényl triméthicone, le benzoate d'alkyle en C12-C15, l'acétate de butyle, l'acétate d'éthyle et la D5 (décaméthylcyclopentasiloxane), à raison d'au moins 50% en poids, à 25°C.

La masse moléculaire moyenne en nombre Mn du polymère avant fonctionnalisation selon l'invention est de préférence comprise entre 1 000 et 3 000 000, de préférence entre 5000 et 1 000 000, mieux entre 8 000 et 500 000. Le poids moléculaire du polymère final va bien entendu varier en fonction du taux de fonctionnalisation (i.e. taux de greffage des OH libres du polycondensat).

De préférence, le taux de greffage des OH libres du polycondensat est compris entre 1 et 80%, notamment entre 2 et 50%, et mieux entre 5 et 25%; toutefois, ce taux peut également être de 100% (la totalité des OH libres est fonctionnalisé par un groupe de jonction).

Le polymère fonctionnalisé selon l'invention peut résulter de l'une des réactions chimiques suivantes :

POL-(OH)n + m OCN-A-NCO → (OH)r POL-OC(O)NH-A-NHC(O)-POL(OH)r'

POL-(OH)n + m' B-NCO → (OH)s POL-OC(O)NH-B

avec m, m' et n : entier supérieur ou égal à 1,
avec de préférence m ≤ n/2 et m' ≤ n; r, r' et s étant de préférence non nuls.

Le polymère formant le squelette polymérique selon l'invention peut être préparé par les procédés d'estérification/polycondensation usuellement employés par l'homme du métier. A titre d'illustration, un procédé général de préparation consiste :
- à mélanger le polyol et les acides monocarboxyliques aromatiques et non aromatiques,
- à chauffer le mélange sous atmosphère inerte, d'abord jusqu'à la température de fusion (généralement 100-130°C) et ensuite à une température comprise entre 150 et 220°C jusqu'à consommation complète des acides monocarboxyliques (atteint lorsque l'indice d'acide est inférieur ou égal à 1), de préférence en distillant au fur et à mesure l'eau formée, puis
- à éventuellement refroidir le mélange à une température comprise entre 90 et 150°C,
- à ajouter l'acide polycarboxylique et/ou l'anhydride cyclique et/ou la lactone, en une seule fois ou de façon séquencée, puis
- à chauffer à nouveau à une température inférieure ou égale à 220°C, notamment comprise entre 170 et 220°C, de préférence en continuant à éliminer l'eau formée, jusqu'à l'obtention des caractéristiques requises en terme d'indice d'acide, de viscosité, d'indice d'hydroxyle et de solubilité.
On obtient ainsi dans un premier temps, le polycondensat qui va former le squelette polymérique.

Il est possible d'ajouter des catalyseurs d'estérification conventionnels, par exemple de type acide sulfonique (notamment à une concentration pondérale comprise entre 1 et 10%) ou type titanate (notamment à une concentration pondérale comprise entre 5 et 100 ppm). Il est également possible de réaliser la réaction, en tout ou en partie, dans un solvant inerte tel que le xylène et/ou sous une pression réduite, pour faciliter l'élimination de l'eau. Avantageusement, on n'utilise ni catalyseur ni solvant.

Ledit procédé de préparation peut comprendre en outre une étape d'addition d'au moins un agent antioxydant dans le milieu réactionnel, notamment à une concentration pondérale comprise entre 0,01 et 1%, par rapport au poids total de monomères, de façon à limiter les éventuelles dégradations liées à un chauffage prolongé. L'agent antioxydant peut être de type primaire ou de type secondaire, et peut être choisi parmi les phénols encombrés, les amines secondaires aromatiques, les composés organophosphorés, les composés soufrés, les lactones, les bisphénols acrylés; et leurs mélanges.

Le polymère final est obtenu par formation d'une liaison uréthanne, entre les fonctions hydroxyle libre du polycondensat décrit ci-dessus et les fonctions isocyanates portées par le groupe de jonction. A titre d'illustration, un procédé général de préparation consiste :
- à chauffer le polycondensat obtenu ci-dessus, comportant au moins une fonction hydroxyle, à une température suffisamment élevée pour pouvoir agiter le polymère sans ajout d'un solvant. Typiquement, cette température peut être comprise entre 80°C et 140°C;
- à rajouter le groupe de jonction portant les fonctions isocyanate;
- à agiter vigoureusement, sous atmosphère contrôlée, ce mélange à une température de l'ordre de 130°C;
- à suivre par spectroscopie infrarouge, la disparition de la bande caractéristique des isocyanates (compris entre 2500 et 2800 cm-1) de manière à arrêter la réaction à la disparition totale du pic, puis à laisser revenir à température ambiante le produit final.
Un solvant peut être utilisé dans le cas où la viscosité du polycondensat initial est trop élevée, même après chauffage à 80-140°C, empêchant une agitation de ce même polycondensat. Il est également possible d'ajouter un catalyseur conventionnel de la formation de liaison uréthane, lorsque la réaction se fait trop difficilement. A titre d'exemple, on peut citer le dilaurate dibutyle étain.

L'utilisation des polymères selon l'invention dans une composition cosmétique conduit, après application de cette composition sur les matières kératiniques, à la formation d'un polymère supramoléculaire.

Par "polymère supramoléculaire", on entend au sens de l'invention, une chaîne ou un réseau polymérique formé de l'assemblage d'un polymère selon l'invention avec au moins un autre polymère selon l'invention, identique ou différent, chaque assemblage comprenant au moins une paire de groupes de jonction appariés, identiques ou différents.

Par "paire de groupes de jonction appariés", on entend au sens de l'invention, deux groupes de jonction dont chacun peut être porté ou non par un même polymère selon l'invention, les deux groupes étant reliés ensemble via au moins trois liaisons H, de préférence au moins 4 liaisons H et plus préférentiellement 4 liaisons H.

Ainsi le polymère supramoléculaire présentera des points de réticulation physique assurés par les liaisons H entre ces paires de groupes de jonction. La réticulation physique assurera le maintien et la persistance de l'effet cosmétique de manière analogue à la réticulation chimique, tout en permettant la réversibilité, c'est-à-dire la possibilité d'éliminer totalement le dépôt.

On a constaté que l'utilisation des polymères selon l'invention peut conduire, après application de la composition sur les matières kératiniques, soit à la formation d'un polymère supramoléculaire sous forme de réseau tridimensionnel réticulé physiquement, se présentant généralement sous forme de film, et ayant une très bonne résistance mécanique, soit à la formation d'un polymère supramoléculaire sous forme d'une longue chaîne polymère, généralement de masse moléculaire élevée résultant de la connection physique des polymères de l'invention.

Les polymères selon l'invention peuvent être utilisés très avantageusement dans une composition cosmétique qui comprend par ailleurs un milieu cosmétiquement acceptable, c'est-à-dire un milieu compatible avec les tissus cutanés comme la peau du visage ou du corps, et les matières kératiniques telles que les cheveux, les cils, les sourcils et les ongles.

La quantité de polymère présent dans les compositions dépend bien entendu du type de composition et des propriétés recherchées et peut varier à l'intérieur d'une gamme très large, comprise généralement entre 0,1 et 80% en poids, de préférence entre 1 et 50% en poids, notamment entre 10 et 45% en poids, voire entre 20 et 40% en poids, et mieux entre 25 et 35% en poids, par rapport au poids de la composition cosmétique finale.

La composition peut alors comprendre, selon l'application envisagée, les constituants habituels à ce type de composition.

La composition selon l'invention peut avantageusement comprendre une phase grasse liquide, qui peut constituer un milieu solvant des polymères selon l'invention, et qui peut comprendre au moins un composé choisi parmi les huiles et/ou solvants d'origine minérale, animale, végétale ou synthétique, carbonés, hydrocarbonés, fluorés et/ou siliconés, volatils ou non volatils, seuls ou en mélange dans la mesure où ils forment un mélange homogène et stable et sont compatibles avec l'utilisation envisagée.

Par 'volatil', on entend au sens de l'invention, tout composé susceptible de s'évaporer au contact des matières kératiniques, ou des lèvres, en moins d'une heure, à température ambiante (25°C) et pression atmosphérique (1 atm). Notamment, ce composé volatil a une pression de vapeur non nulle, à température ambiante et pression atmosphérique, notamment allant de 0,13 Pa à 40 000 Pa (10⁻³ à 300 mm de Hg), en particulier allant de 1,3 Pa à 13 000 Pa (0,01 à 100 mm de Hg), et plus particulièrement allant de 1,3 Pa à 1300 Pa (0,01 à 10 mm de Hg).

Par opposition, on entend par 'non volatil', un composé restant sur les matières kératiniques ou les lèvres à température ambiante et pression atmosphérique, au moins une heure et ayant notamment une pression de vapeur inférieure à 10⁻³ mm de Hg (0,13Pa).

De préférence, le milieu physiologiquement acceptable de la composition selon l'invention peut comprendre, dans une phase grasse liquide, au moins une huile et/ou un solvant qui peut être choisi parmi, seul ou en mélange :
1/ les esters des acides monocarboxyliques avec les monoalcools et polyalcools; avantageusement, ledit ester est un benzoate d'alkyle en C12-C15 ou répond à la formule suivante : R'₁-COO-R'₂ où :
   R'₁ représente un radical alkyle linéaire ou ramifié de 1 à 40 atomes de carbone, de préférence de 7 à 19 atomes de carbone, comprenant éventuellement une ou plusieurs double liaisons éthyléniques, éventuellement substitué et dont la chaîne hydrocarbonée peut être interrompue par un ou plusieurs hétéroatomes choisis parmi N et O et/ou une ou plusieurs fonctions carbonyles, et
   R'₂ représente un radical alkyle linéaire ou ramifié de 1 à 40 atomes de carbone, de préférence de 3 à 30 atomes de carbone et mieux de 3 à 20 atomes de carbone, comprenant éventuellement une ou plusieurs double liaisons éthyléniques, éventuellement substitué et dont la chaîne hydrocarbonée peut être interrompue par un ou plusieurs hétéroatomes choisis parmi N et O et/ou une ou plusieurs fonctions carbonyles.

   Par "éventuellement substitué", on entend que R'₁ et/ou R'₂ peuvent porter un ou plusieurs substituants choisis, par exemple, parmi les groupements comprenant un ou plusieurs hétéroatomes choisi parmi O et/ou N, tels que amino, amine, alcoxy, hydroxyle.
   Des exemples des groupes R'₁ sont ceux dérivés des acides gras de préférence supérieur choisis dans le groupe constitué des acides acétique, propionique, butyrique, caproïque, caprylique, pélargonique, caprique, undécanoïque, laurique, myristique, palmitique, stéarique, isostéarique, arachidique, béhénique, oléique, linolénique, linoléïque, oléostéarique, arachidonique, érucique, et de leurs mélanges.
   De préférence, R'₁ est un groupe alkyle ramifié non substitué de 4 à 14 atomes de carbone, de préférence de 8 à 10 atomes de carbone et R₂ est un groupe alkyle ramifié non substitué de 5 à 15 atomes de carbone, de préférence de 9 à 11 atomes de carbone.
   On peut en particulier citer, de préférence, les esters en C₈-C₄₈, éventuellement incorporant dans leur chaîne hydrocarbonée un ou plusieurs hétéroatomes parmi N et O et/ou une ou plusieurs fonctions carbonyle; et plus particulièrement l'huile de purcellin (octanoate de cétostéaryle), l'isononanoate d'isononyle, le myristate d'isopropyle, le palmitate d'isopropyle, le palmitate d'éthyl-2-hexyle, le stéarate d'octyl-2-dodécyle, l'érucate d'octyl-2-dodécyle, l'isostéarate d'isostéaryle, le benzoate d'alcool en C₁₂ à C₁₅, le laurate d'hexyle, l'adipate de diisopropyle; et les heptanoates, octanoates, décanoates ou ricinoléates d'alcools ou de polyalcools, par exemple d'alcools gras comme le dioctanoate de propylène glycol, ainsi que le N-lauroyl sarcosinate d'isopropyle (notamment Eldew-205SL d'Ajinomoto); les esters hydroxylés comme le lactate d'isostéaryle, le malate de di-isostéaryle; et les esters du pentaérythritol; les esters ramifiés en C8-C16, notamment le néo-pentanoate d'isohexyle.
2/ les huiles végétales hydrocarbonées à forte teneur en triglycérides constitués d'esters d'acides gras et de glycérol dont les acides gras peuvent avoir des longueurs de chaînes variées de C₄ à C₂₄, ces dernières pouvant être linéaires ou ramifiées, saturées ou insaturées; ces huiles sont notamment les huiles de germe de blé, de maïs, de tournesol, de karité, de ricin, d'amandes douces, de macadamia, d'abricot, de soja, de colza, de coton, de luzerne, de pavot, de potimarron, de sésame, de courge, d'avocat, de noisette, de pépins de raisin ou de cassis, d'onagre, de millet, d'orge, de quinoa, d'olive, de seigle, de carthame, de bancoulier, de passiflore, de rosier muscat, de jojoba, de palme, de calophyllum; ou encore les triglycérides des acides caprylique/caprique comme ceux vendus par la société Stearinerie Dubois ou ceux vendus sous les dénominations "Miglyol 810^{®}" , "812^{®} " et "818^{®} " par la société Dynamit Nobel.
3/ les alcools, et notamment les monoalcools, en C6-C32, notamment C12-C26, comme l'alcool oléïque, l'alcool linoléique, l'alcool linolénique, l'alcool isostéarylique, le 2-hexyldécanol, le 2-butyloctanol, le 2-undécylpentadécanol et l'octyldodécanol;
4/ les huiles hydrocarbonées, linéaires ou ramifiés, volatiles ou non, d'origine synthétique ou minérale, qui peuvent être choisies parmi les huiles hydrocarbonées ayant de 5 à 100 atomes de carbones, et notamment la vaseline, les polydécènes, les polyisobutènes hydrogénés tel que le Parléam, le squalane, le perhydrosqualène et leurs mélanges.
   On peut plus particulièrement citer les alcanes linéaires, ramifiés et/ou cycliques en C5-C48, et préférentiellement les alcanes ramifiés en C8-C16 comme les isoalcanes en C8-C16 d'origine pétrolière (appelées aussi isoparaffines); notamment le décane, l'heptane, le dodécane, le cyclohexane; ainsi que l'isododécane, l'isodécane, l'isohexadécane.
5/ les huiles de silicone, volatiles ou non volatiles;
   Comme huiles de silicone volatiles, on peut citer les huiles de silicones linéaires ou cycliques volatiles, notamment celles ayant une viscosité inférieure à 8 centistokes, et ayant notamment de 2 à 10 atomes de silicium, ces silicones comportant éventuellement des groupes alkyles ou alkoxy ayant de 1 à 22 atomes de carbone; et en particulier l'octaméthylcyclotétrasiloxane, le décaméthylcyclopentasiloxane, le dodécaméthylcyclohexasiloxane, l'heptaméthylhexyltrisiloxane, l'heptaméthyloctyltrisiloxane, l'hexaméthyldisiloxane, l'octaméthyltrisiloxane, le décaméthyltétrasiloxane, le dodécaméthylpentasiloxane, le méthylhexyldiméthylsiloxane et leurs mélanges.
   Les huiles de silicone non volatiles utilisables selon l'invention peuvent être les polydiméthylsiloxanes (PDMS), les polydiméthylsiloxanes comportant des groupements alkyle ou alcoxy, pendant et/ou en bout de chaîne siliconée, groupements ayant chacun de 2 à 24 atomes de carbone, les silicones phénylées comme les phényltriméthicones, les phényldiméthicones, les phényltriméthylsiloxydiphénylsiloxanes, les diphényldiméthicones, les diphénylméthyldiphényltrisiloxanes, les 2-phényléthyl triméthylsiloxysilicates.

Préférentiellement, le milieu physiologiquement acceptable de la composition selon l'invention comprend, dans une phase grasse liquide, au moins une huile et/ou un solvant choisi parmi, seul ou en mélange, l'isododécane, le Parléam, l'isononanoate d'isononyle, l'octyldodécanol, la phényl triméthicone, les benzoates d'alkyle en C12-C15, les acétates de butyle et d'éthyle, et/ou la D5 (décaméthylcyclopentasiloxane).

La phase grasse liquide peut en outre comprendre des huiles et/ou solvants additionnels, qui peuvent être choisis parmi, seul ou en mélange :
- les huiles fluorées telles que les perfluoropolyéthers, les perfluoroalcanes comme la perfluorodécaline, les perfluorodamantanes, les monoesters, diesters et triesters de perfluoroalkylphosphates et les huiles esters fluorés;
- les huiles d'origine animale;
- les éthers en C₆ à C₄₀, notamment en C10-C40; les éthers de propylène glycol liquides à température ambiante tels que le monométhyléther de propylène glycol, l'acétate de monométhyléther de propylène glycol, le mono n-butyl éther de dipropylène glycol ;
- les acides gras en C₈-C₃₂, comme l'acide oléique, l'acide linoléique, l'acide linolénique et leurs mélanges.
- les huiles bifonctionnelles, comprenant deux fonctions choisies parmi ester et/ou amide et comprenant de 6 à 30 atomes de carbone, notamment 8 à 28 atomes de carbone, mieux de 10 à 24 carbones, et 4 hétéroatomes choisis parmi O et N; de préférence les fonctions amide et ester étant dans la chaîne;
- les cétones liquides à température ambiante (25°C) tels que la méthyléthylcétone, la méthylisobutylcétone, la diisobutylcétone, l'isophorone, la cyclohexanone, l'acétone ;
- les aldéhydes liquides à température ambiante tels que le benzaldéhyde, l'acétaldéhyde;

La phase grasse liquide peut représenter 1 à 90% en poids de la composition, notamment de 5 à 75% en poids, en particulier de 10 à 60% en poids, voire de 25 à 55% en poids, du poids total de la composition.

La composition selon l'invention peut comprendre avantageusement un agent épaississant qui peut en particulier être choisi parmi :
- les silices notamment hydrophobes, telles que celles décrites dans le document EP-A-898960, et par exemple commercialisées sous les références "AEROSIL R812^{®}" par la société Degussa, "CAB-O-SIL TS-530^{®}", "CAB-O-SIL TS-610^{®}", "CAB-O-SIL TS-720^{®}" par la société Cabot, "AEROSIL R972^{®}", "AEROSIL R974^{®}" par la société Degussa ;
- les argiles telles que la montmorillonite, les argiles modifiées telles que les bentones par exemple, l'hectorite stéaralkonium, la bentonite stéaralkonium,
- les alkyléther de polysaccharides (notamment dont le groupe alkyle comporte de 1 à 24 atomes de carbones, de préférence de 1 à 10, mieux de 1 à 6, et plus spécialement de 1 à 3) tels que ceux décrits dans le document EP-A-898958.

La quantité d'agent épaississant dans la composition selon l'invention peut aller de 0,05 à 40% en poids, par rapport au poids total de la composition, de préférence de 0,5 à 20% et mieux de 1 à 15% en poids.

La composition selon l'invention peut également comprendre au moins une cire d'origine végétale, animale, minérale ou de synthèse, voire siliconée.

On peut en particulier citer, seules ou en mélange, les cires hydrocarbonées telles que la cire d'abeilles; la cire de Carnauba, de Candellila, d'Ouricoury, du Japon, les cires de fibres de lièges ou de canne à sucre; les cires de paraffine, de lignite; les cires microcristallines; la cire de lanoline; la cire de Montan; les ozokérites; les cires de polyéthylène; les cires obtenues par synthèse de Fischer-Tropsch; les huiles hydrogénées, les esters gras et les glycérides concrets à 25°C. On peut également utiliser des cires de silicone, parmi lesquelles on peut citer les alkyls, alcoxys et/ou esters de polyméthylsiloxane.

La quantité de cire dans la composition selon l'invention peut aller de 0,1 à 70% en poids, par rapport au poids total de la composition, de préférence de 1 à 40% en poids, et mieux de 5 à 30% en poids.

La composition selon l'invention peut également comprendre une ou plusieurs matières colorantes choisies parmi les composés pulvérulents comme les pigments, les charges, les nacres et les paillettes, et/ou les colorants liposolubles ou hydrosolubles.

Les matières colorantes, notamment pulvérulentes, peuvent être présentes, dans la composition, en une teneur de 0,01 à 50% en poids, par rapport au poids de la composition, de préférence de 0,1 à 40% en poids, voire de 1 à 30% en poids. Par pigments, il faut comprendre des particules de toute forme, blanches ou colorées, minérales ou organiques, insolubles dans le milieu physiologique, destinées à colorer la composition.

Par nacres, il faut comprendre des particules de toute forme irisées, notamment produites par certains mollusques dans leur coquille ou bien synthétisées.

Les pigments peuvent être blancs ou colorés, minéraux et/ou organiques, interférentiels ou non. On peut citer, parmi les pigments minéraux, le dioxyde de titane, éventuellement traité en surface, les oxydes de zirconium ou de cérium, ainsi que les oxydes de fer ou de chrome, le violet de manganèse, le bleu outremer, l'hydrate de chrome et le bleu ferrique. Parmi les pigments organiques, on peut citer le noir de carbone, les pigments de type D & C, et les laques à base de carmin de cochenille, de baryum, strontium, calcium, aluminium.

Les pigments nacrés peuvent être choisis parmi les pigments nacrés blancs tels que le mica recouvert de titane, ou d'oxychlorure de bismuth, les pigments nacrés colorés tels que le mica titane avec des oxydes de fer, le mica titane avec notamment du bleu ferrique ou de l'oxyde de chrome, le mica titane avec un pigment organique du type précité ainsi que les pigments nacrés à base d'oxychlorure de bismuth.

Les charges peuvent être minérales ou organiques, lamellaires ou sphériques. On peut citer le talc, le mica, la silice, le kaolin, les poudres de Nylon et de polyéthylène, de poly-β-alanine et de polyéthylène, le Téflon, la lauroyl-lysine, l'amidon, le nitrure de bore, les poudres de polymères de tétrafluoroéthylène, les microsphères creuses telles que l'Expancel (Nobel Industrie), le polytrap (Dow Corning) et les microbilles de résine de silicone (Tospearls de Toshiba, par exemple), le carbonate de calcium précipité, le carbonate et l'hydro-carbonate de magnésium, l'hydroxyapatite, les microsphères de silice creuses (SILICA BEADS de MAPRECOS), les microcapsules de verre ou de céramique, les savons métalliques dérivés d'acides organiques carboxyliques ayant de 8 à 22 atomes de carbone, de préférence de 12 à 18 atomes de carbone, par exemple le stéarate de zinc, de magnésium ou de lithium, le laurate de zinc, le myristate de magnésium. Les colorants liposolubles sont par exemple le rouge Soudan, le DC Red 17, le DC Green 6, le β-carotène, l'huile de soja, le brun Soudan, le DC Yellow, 11, le DC Violet 2, le DC orange 5, le jaune quinoléine. Ils peuvent représenter 0,01 à 20% du poids de la composition et mieux de 0,1 à 6 %.

Les colorants hydrosolubles sont par exemple le jus de betterave, le bleu de méthylène et peuvent représenter 0,01 à 6% du poids total de la composition.

La composition peut comprendre, en outre, d'autres ingrédients utilisés couramment dans les compositions cosmétiques. De tels ingrédients peuvent être choisis parmi les antioxydants, les parfums, les huiles essentielles, les conservateurs, les actifs cosmétiques, les hydratants, les vitamines, les céramides, les filtres solaires, les tensioactifs, les gélifiants, les agents d'étalement, les agents mouillants, les agents dispersants, les anti-mousses, les neutralisants, les stabilisants, les polymères et notamment les polymères filmogènes liposolubles, et leurs mélanges.

Bien entendu, l'homme du métier veillera à choisir ce ou ces éventuels composés complémentaires, et/ou leur quantité, de manière telle que les propriétés avantageuses de la composition pour l'utilisation selon l'invention ne soient pas, ou substantiellement pas, altérées par l'adjonction envisagée.

Les compositions selon l'invention peuvent se présenter sous toute forme acceptable et usuelle pour une composition cosmétique. Elles peuvent donc se présenter sous la forme d'une suspension, une dispersion notamment d'huile dans de l'eau grâce à des vésicules; une solution organique ou huileuse éventuellement épaissie voire gélifiée; une émulsion huile-dans-eau, eau-dans-huile, ou multiple; un gel ou une mousse; un gel huileux ou émulsionné; une dispersion de vésicules notamment lipidiques; une lotion biphase ou multiphase; un spray; d'une lotion, d'une crème, d'une pommade, d'une pâte souple, d'un onguent, d'un solide coulé ou moulé et notamment en stick ou en coupelle, ou encore de solide compacté.

L'homme du métier pourra choisir la forme galénique appropriée, ainsi que sa méthode de préparation, sur la base de ses connaissances générales, en tenant compte d'une part de la nature des constituants utilisés, notamment de leur solubilité dans le support, et d'autre part de l'application envisagée pour la composition.

Les compositions conformes à l'invention présentant une brillance et une tenue de ladite brillance améliorées par rapport à l'état de l'art, elles peuvent être utilisées pour le soin ou le maquillage des matières kératiniques telles que les cheveux, la peau, les cils, les sourcils, les ongles, les lèvres, le cuir chevelu et plus particulièrement pour le maquillage des lèvres, des cils et/ou du visage.

Elles peuvent donc se présenter sous la forme d'un produit de soin et/ou de maquillage de la peau du corps ou du visage, des lèvres, des cils, des sourcils, des cheveux, du cuir cheveu ou des ongles; d'un produit solaire ou autobronzant; d'un produit capillaire notamment de coloration, de conditionnement et/ou de soin des cheveux; elles se présentent avantageusement sous forme de composition de maquillage, notamment de mascara, d'eye-liner, de rouge à lèvres, de brillant à lèvres (gloss), de fard à joues ou à paupières, de fond de teint, de vernis à ongles ou de soin des ongles.

L'invention a encore pour objet un procédé de traitement cosmétique des matières kératiniques, notamment de la peau du corps ou du visage, des lèvres, des ongles, des cheveux et/ou des cils, comprenant l'application sur lesdites matières d'une composition cosmétique telle que définie précédemment.

Ce procédé selon l'invention permet notamment le soin ou le maquillage desdites matières kératiniques, en particulier des lèvres et/ou des ongles, par application d'une composition notamment de rouge à lèvres ou de brillant à lèvres (gloss) ou de vernis à ongles selon l'invention.

L'invention est illustrée plus en détail dans les exemples suivants.

### Méthode de mesure de la viscosité

La viscosité à 125°C du polymère est mesurée à l'aide d'un viscosimètre à cône plan de type BROOKFIELD CAP 1000+.

Le cône-plan adapté est déterminé par l'homme du métier, sur la base de ses connaissances; notamment :
- entre 50 et 500 mPa.s, on peut utiliser un cône 02
- entre 500 et 1000 mPa.s : cône 03 ou 05 (pour les valeurs hautes)
- entre 1000 et 4000 mPa.s : cône 05
- entre 4000 et 10000 mPa.s : cône 06

De préférence, on utilise dans la mesure du possible, le même cône lorsque l'on souhaite comparer deux polymères.

### Exemple 1

### A/ Synthèse du pentaérythrityl benzoate/isophtalate/isostéarate

Dans un réacteur équipé d'une agitation mécanique, d'une arrivée d'argon et d'un système de distillation, on charge 20 g d'acide benzoïque, 280 g d'acide isostéarique et 100 g de pentaérythritol, puis on chauffe progressivement, sous un léger courant d'argon, à 110-130°C pour obtenir une solution homogène. On augmente ensuite progressivement la température jusqu'à 180°C et on la maintient pendant environ 2 heures. On augmente de nouveau la température jusqu'à 220°C et on la maintient jusqu'à ce qu'on obtienne un indice d'acide inférieur ou égal à 1, ce qui prend environ 11 heures. On refroidit à une température comprise entre 100 et 130°C puis on introduit 100 g d'acide isophtalique et on chauffe à nouveau progressivement jusqu'à 220°C pendant environ 11 heures.

On obtient ainsi 405 g de polycondensat pentaérythrityl benzoate/isophtalate/isostéarate sous la forme d'une huile très épaisse.

Le polycondensat présente un indice d'hydroxyle égal à 72.

### B/ Synthèse du polymère fonctionnalisé par des uréidopyrimidones

On chauffe à 110°C, 100 g du polycondensat préparé ci-dessus, puis on ajoute 3,22 g (10 mmoles) de dendrons uréidopyrimidone monofonctionnalisés isocyanate de structure :

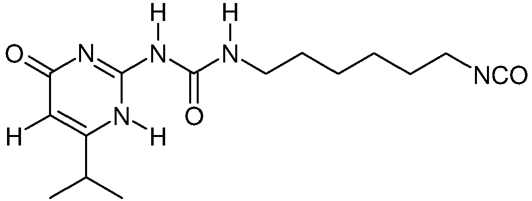

Le mélange est agité et chauffé sous argon à 130°C pendant 15 minutes. La réaction est stoppée lorsque le pic correspondant à l'isocyanate (2250 cm⁻¹) en spectroscopie infrarouge a disparu.

On obtient le polymère final sous forme d'une huile visqueuse. Le rendement est quantitatif. Le polymère final possède un indice hydroxyle de 59, ce qui correspond à un taux de fonctionnalisation de 20%.

### C/ Mesure de la brillance et de la viscosité

On mesure la brillance du polymère ci-dessus, après étalement d'un film de 25 µm sur bioskin noir, après 10 minutes sur plaque à 35°C, avec un brillancemètre micro-trigloss. Le polymère est véhiculé dans l'isododécane à un extrait sec permettant d'obtenir après évaporation du solvant, un film de 25 microns. On obtient les résultats suivants :

| | Viscosité | Brillance |
|---|---|---|
| | (125°C) | Angle 20°, 60° et 85° |
| Polymère selon l'invention (obtenu à l'étape B) | 1800 mPa.s | 73, 84, 81 |
| | Cône 5 | |
| Polycondensat obtenu à l'étape A | 700 mPa.s | 73, 84, 84 |
| | Cône 5 | |

On constate que la fonctionnalisation par des ureïdopyrimidones conduit à un polymère de viscosité plus élevée, donc de meilleure tenue, tout en gardant la brillance du film. De plus, le polymère selon l'invention conserve sa solubilité dans les huiles cosmétiques couramment utilisées (isododécane notamment).

### Exemple 2

On chauffe à 100°C, 49,2 g du polycondensat préparé à l'étape A de l'exemple 1 ci-dessus, puis on ajoute 4,05 g de dendrons uréidopyrimidone monofonctionnalisés isocyanate de structure :

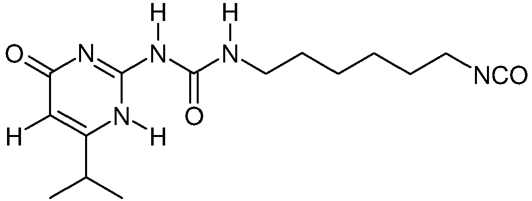

Le mélange est agité et chauffé sous argon à 130°C pendant 30 minutes.

On obtient le polymère final sous forme d'une huile solide. Le polymère véhiculé dans une huile cosmétique (isododécane) permet d'obtenir un film, après application de la solution et évaporation du solvant. La brillance du film est équivalente à celle du polyester avant fonctionnalisation.

### Exemple 3

On chauffe à 100°C, 43,7 g du polycondensat préparé à l'étape A de l'exemple 1 ci-dessus, puis on ajoute 5,35 g de dendrons uréidopyrimidone monofonctionnalisés isocyanate de structure :

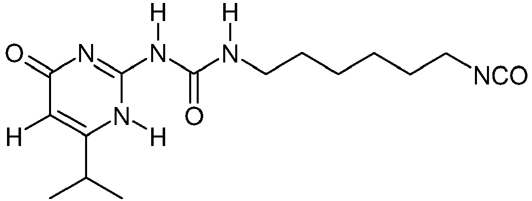

Le mélange est agité et chauffé sous argon à 130°C pendant 30 minutes.

On obtient le polymère final sous forme d'une huile solide. Le polymère véhiculé dans une huile cosmétique (isododécane) permet d'obtenir un film, après application de la solution et évaporation du solvant. La brillance du film est équivalente à celle du polyester avant fonctionnalisation.

### Exemple 4

### A/

On introduit 720 g d'acide benzoïque, 1392 g d'acides gras d'huile de ricin (NOURACID DE 655 d'Akzo Nobel) et 916,8 g de pentaérythritol, dans un réacteur de 2 litres. Le réacteur est maintenu sous atmosphère d'argon durant toute la synthèse (bullage). Le mélange est chauffé à 130°C pour obtenir une solution homogène, ensuite il est maintenu à 180°C pendant 1 heure. On augmente la température à 230°C jusqu'à un indice d'acide inférieur ou égal à 1.

On descend la température à 160°C et on ajoute 969,6 g d'anhydride phtalique, puis on chauffe à 230°C. A partir de 4 heures, on suit la cinétique de la polymérisation par son indice d'acide. Quand on obtient un indice d'acide inférieur à 25, on arrête la synthèse.

On obtient un polymère ayant un indice d'acide de 23,1 et un indice d'hydroxyle de 165.

### B/

On dissout 29,6 g du polyester synthétisé ci-dessus dans 50ml de chloroforme en présence du catalyseur dilaurate dibutyle étain. On ajoute 1,16 g de l'uréidopyrimidone représenté ci-dessous au milieu réactionnel :

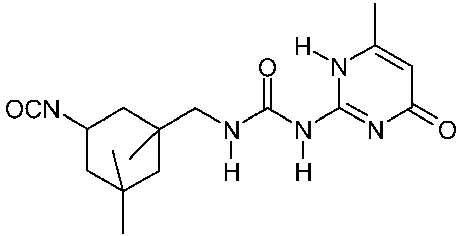

Le mélange est agité sous argon, à 60°C pendant 16 heures. Le mélange réactionnel est ensuite précipité dans 300 ml d'hexane, le précipité ainsi obtenu est séché sous vide, et on obtient le produit voulu sous forme d'un solide jaune.

Ce solide jaune est soluble à 25°C, 50% en poids, dans des solvants comme l'acétate de butyle et permet d'obtenir une solution de polymère dans un solvant cosmétique.

### Exemple 5

On dissout 29 g du polyester synthétisé à l'étape A de l'exemple 4, dans 60ml de chloroforme en présence du catalyseur dilaurate dibutyle étain. On ajoute 2,91 g de l'uréidopyrimidone représenté ci-dessous au milieu réactionnel :

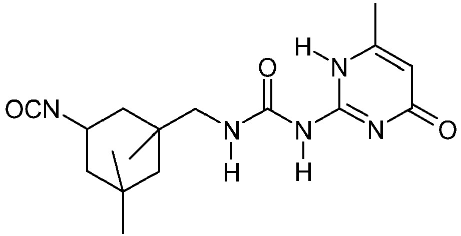

Le mélange est agité sous argon, à 60°C pendant 16 heures. Le mélange réactionnel est ensuite précipité dans 300ml d'hexane, le précipité ainsi obtenu est séché sous vide, et on obtient le produit voulu sous forme d'un solide cassant jaune. Ce solide jaune est soluble à 25°C, 50% en poids, dans des solvants comme l'acétate de butyle et permet d'obtenir une solution de polymère dans un solvant cosmétique.

### Exemple 6

On dissout 4,38 g du polyester synthétisé à l'étape A de l'exemple 4, dans 20ml de chloroforme en présence de dilaurate dibutyle étain. On ajoute 0,87 g de l'uréidopyrimidone représenté ci-dessous au milieu réactionnel :

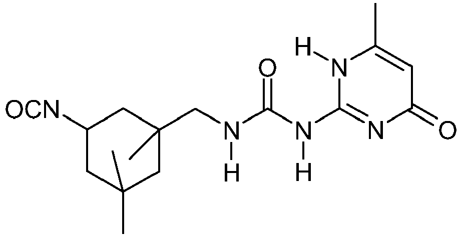

Le mélange est agité sous argon, à 60°C pendant 16 heures. Le mélange réactionnel est ensuite précipité dans 150ml d'hexane, le précipité ainsi obtenu est séché sous vide, et on obtient le produit voulu sous forme d'un solide cassant jaune. Ce solide jaune est soluble à 25°C, 50% en poids, dans des solvants comme l'acétate de butyle et permet d'obtenir une solution de polymère dans un solvant cosmétique.

### Exemple 7

### A/

On introduit 90 g d'acide benzoïque, 160 g d'acide isostéarique et 130 g de pentaérythritol, dans un réacteur de 0,5 litres. Le réacteur est maintenu sous atmosphère d'argon durant toute la synthèse (bullage). Le mélange est chauffé à 130°C pour obtenir une solution homogène, ensuite il est maintenu à 180°C pendant 1 heure. On augmente la température à 230°C jusqu'à un indice d'acide inférieur ou égal à 1.

On descend la température à 160°C et on ajoute 135 g d'acide isophtalique, puis on chauffe à 230°C. A partir de 4 heures, on suit la cinétique de la polymérisation par son indice d'acide. Quand on obtient un indice d'acide inférieur à 10, on arrête la synthèse.

On obtient un polymère ayant un indice d'acide de 8,0 et un indice d'hydroxyle de 165.

### B/

On dissout 12 g du polyester synthétisé ci-dessus dans 50ml de chloroforme en présence de dilaurate dibutyle étain. On ajoute 0,61 g de l'uréidopyrimidone représenté ci-dessous au milieu réactionnel :

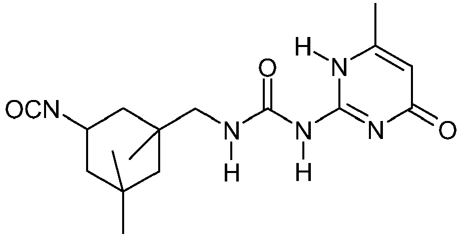

Le mélange est agité sous argon, à 60°C pendant 16 heures. Le mélange réactionnel est ensuite précipité dans 150 ml d'hexane, le précipité ainsi obtenu est séché sous vide, et on obtient le produit voulu sous forme d'un solide cassant jaune.

### Exemple 8

On dissout 10,5 g du polyester synthétisé à l'étape A de l'exemple 7, dans 50ml de chloroforme en présence de dilaurate dibutyle étain. On ajoute 1,07 g de l'uréidopyrimidone représenté ci-dessous au milieu réactionnel :

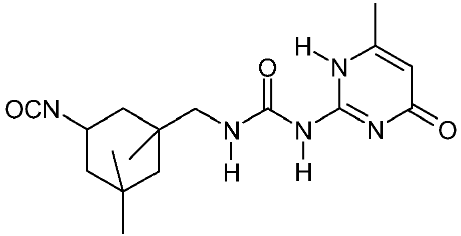

Le mélange est agité sous argon, à 60°C pendant 16 heures. Le mélange réactionnel est ensuite précipité dans 150ml d'hexane, le précipité ainsi obtenu est séché sous vide, et on obtient le produit voulu sous forme d'un solide cassant jaune.

### Exemple 9

On prépare un stick de rouge à lèvres comprenant :
- 15% de cire de polyéthylène
- 30% de polymère préparé à l'exemple 1
- 50% d'isoparaffine
- 5% de pigment DC Red7

### Exemple 10

On prépare un gloss comprenant :
- 30% de polybutène (monooléfines/isoparaffines 95/5, PM 2060)
- 35% de polybutène (monoolefines/isoparaffines PM=920)
- 30% de polymère préparé à l'exemple 1
- 5% de pigment DC red 7.

### Exemple 11

On prépare un vernis à ongles comprenant (% en poids):

| | |
|---|---|
| - Nitrocellulose | 15 % |
| - polymère de l'exemple 6 | 9 % |
| - Acétyl citrate de tributyle | 5 % |
| - pigments | 1 % |
| - Hectorite | 1,2 % |
| - Alcool Isopropylique | 8 % |
| - Acétate d'éthyle, acétate de butyle | qsp 100% |

## Revendications

1. Composition cosmétique ou dermatologique comprenant, dans un milieu cosmétiquement ou dermatologiquement acceptable, un polymère comportant :
(a) un squelette polymérique susceptible d'être obtenu par réaction :
- de 10 à 60% en poids, par rapport au poids total dudit squelette, d'au moins un polyol comprenant 3 à 6 groupes hydroxyles;
- de 0,1 à 80% en poids, par rapport au poids total dudit squelette, d'un mélange d'acide monocarboxylique aromatique comprenant de 6 à 32 atomes de carbone et d'acide monocarboxylique non aromatique comprenant de 6 à 32 atomes de carbone ;
- de 5 à 60% en poids, par rapport au poids total dudit squelette, d'au moins un acide polycarboxylique comprenant au moins 2 groupes carboxyliques COOH, et/ou un anhydride cyclique d'un tel acide polycarboxylique;
et
(b) au moins un groupe de jonction lié audit squelette polymérique et capable d'établir des liaisons H avec un ou plusieurs groupes de jonction partenaires, chaque appariement d'un groupe de jonction faisant intervenir au moins 3 liaisons H (hydrogène),
ledit squelette étant lié au groupe de jonction par au moins une fonction uréthanne,
le groupe de jonction porteurs de groupes isocyanates étant de formule OCN-A-NCO, le radical divalent A étant de structure : 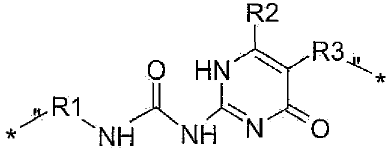 dans laquelle :
- R1 et R3, identiques ou différents, représentent un radical carboné divalent choisi parmi (i) un groupe alkyle linéaire ou ramifié en C₁-C₃₀, (ii) un groupe cycloalkyle en C₄-C₁₂ et (iii) un groupe aryle en C₄-C₁₂; comprenant éventuellement 1 à 8 hétéroatomes choisis parmi O, N, S, F, Si et P; et/ou éventuellement substitué par une fonction ester, amide ou par un radical alkyle en C₁-C₁₂; ou un mélange de ces groupes;
- R2 représente H, halogène (Br, Cl, F) ou un groupe hydrocarboné monovalent, linéaire, ramifié ou cyclique, en C1-C32, saturé ou non, éventuellement aromatique, pouvant contenir un ou plusieurs hétéroatomes choisis parmi O, S, N, P, F.
ou de formule B-NCO, le radical monovalent B étant dé formule : 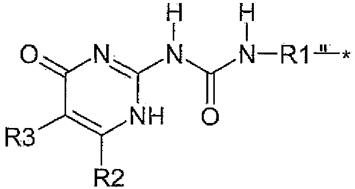 dans laquelle :
- le radical R₁ représentant un groupe carboné divalent choisi parmi (i) un groupe alkyle linéaire ou ramifié en C₁-C₃₀, (ii) un groupe cycloalkyl en C₄-C₁₂ et (iii) un groupe aryle en C₄-C₁₂; comprenant éventuellement 1 à 8 hétéroatomes choisis parmi O, N, S, F, Si et P; et/ou éventuellement substitué par une fonction ester, amide ou par un radical alkyle en C₁-C₁₂; ou un mélange de ces groupes;
- les radicaux R2 et R3, identiques ou différents, représentent un atome d'hydrogène ou un radical carboné, notamment hydrocarboné (alkyle), linéaire, ramifié ou cyclique, saturé ou insaturé, en C1-C32, pouvant comprendre un ou plusieurs hétéroatomes choisis parmi O N, S, F, Si et P.

2. Composition selon la revendication 1, dans laquelle le polyol est choisi parmi, seul ou en mélange:
- les triols, tels que 1,2,4-butanetriol, le 1,2,6-hexanetriol, le triméthyloléthane, le triméthylolpropane, le glycérol;
- les tétraols, tels que le pentaérythritol, l'érythritol, le diglycérol ou le ditriméthylolpropane;
- les pentols tels que le xylitol,
- les hexols tels que le sorbitol et le mannitol; ou encore le dipentaérythritol ou le triglycérol.

3. Composition selon l'une des revendications précédentes, dans laquelle le polyol est le pentaérythritol.

4. Composition selon l'une des revendications précédentes, dans laquelle le polyol, ou le mélange de polyols, représente 10 à 60% en poids, notamment 12 à 40% en poids, et mieux 14 à 25% en poids, du poids total du polycondensat formant le squelette polymérique.

5. Composition selon l'une des revendications précédentes, dans laquelle le polyol, ou le mélange de polyols, représente 10 à 25% en poids du poids total du polycondensat formant le squelette polymérique.

6. Composition selon l'une des revendications précédentes, dans laquelle l'acide monocarboxylique aromatique est choisi parmi, seul ou en mélange:
l'acide benzoïque, l'acide o-toluique, l'acide m-toluique, l'acide p-toluique, l'acide 1-naphtoïque, l'acide 2-naphtoïque, l'acide 4-tert-butyl-benzoïque, l'acide 1-méthyl-2-naphtoïque, l'acide 2-isopropyl-1-naphtoïque.

7. Composition selon l'une des revendications précédentes, dans laquelle l'acide monocarboxylique aromatique est l'acide benzoïque.

8. Composition selon l'une des revendications précédentes, dans laquelle l'acide monocarboxylique non aromatique est choisi parmi, seul ou en mélange:
- les acides monocarboxyliques saturés tels que l'acide caproïque, l'acide caprylique, l'acide isoheptanoïque, l'acide 4-éthylpentanoïque, l'acide 2-éthylhexanoïque, l'acide 4,5-diméthylhexanoïque, l'acide 2-heptylheptanoïque, l'acide 3,5,5-triméthylhexanoïque, l'acide octanoïque, l'acide isooctanoïque, l'acide nonanoïque, l'acide décanoïque, l'acide isononanoïque, l'acide laurique, l'acide tridécanoïque, l'acide myristique, l'acide palmitique, l'acide stéarique, l'acide isostéarique, l'acide arachidique, l'acide béhénique, l'acide cérotique (hexacosanoïque); l'acide cyclopentanecarboxylique, l'acide cyclopentaneacétique, l'acide 3-cyclopentylpropionique, l'acide cyclohexanecarboxylique, l'acide cyclohexylacétique, l'acide 4-cyclohexylbutyrique;
- les acides monocarboxyliques insaturés non aromatiques, tels que l'acide caproléique, l'acide obtusilique, l'acide undécylénique, l'acide dodécylénique, l'acide lindérique, l'acide myristoléique, l'acide physétérique, l'acide tsuzuique, l'acide palmitoléique, l'acide oléique, l'acide pétrosélinique, l'acide vaccénique, l'acide élaidique, l'acide gondoïque, l'acide gadoléique, l'acide érucique, l'acide cétoléique, l'acide nervonique, l'acide oléïque, l'acide linoléique, l'acide linolénique, l'acide arachidonique, l'acide ruménique, l'acide éicosapentaènoïque, l'acide docosahexaènoïque;
- les acides conjugués tels que l'acide stillinguique (10:2 2t,4c), l'acide ruménique (18:2, 9c,11t), l'acide linoléique, conjugué (18:2 10t,12c), l'acide linolénique conjugué (18:3 9c,11t,15c), l'acide linolénique conjugué (18:3 6c,9c,11t) et l'acide alpha-éléostéarique (18:3 9c,11t,13t), seuls ou en mélange.

9. Composition selon l'une des revendications précédentes, dans laquelle l'acide monocarboxylique non aromatique est choisi parmi l'acide laurique, l'acide palmitique, l'acide stéarique, l'acide béhénique, l'acide 2-éthylhexanoïque, l'acide isooctanoïque, l'acide isononanoïque, l'acide isostéarique, l'acide caproléique, l'acide obtusilique, l'acide undécylénique, l'acide dodécylénique, l'acide lindérique, l'acide myristoléique, l'acide physétérique, l'acide tsuzuique, l'acide palmitoléique, l'acide oléique, l'acide linoléique, l'acide linolénique, l'acide arachidonique, et leurs mélanges.

10. Composition selon l'une des revendications précédentes, dans laquelle le mélange d'acides monocarboxyliques représente 0,1 à 80% en poids, notamment 0,5 à 70% en poids, voire 1 à 65% en poids, et mieux 1,5 à 60% en poids, du poids total du polycondensat formant le squelette polymérique.

11. Composition selon l'une des revendications précédentes, dans laquelle le mélange d'acides monocarboxyliques représente 1,5 à 60% en poids, du poids total du polycondensat formant le squelette polymérique.

12. Composition selon l'une des revendications précédentes, dans laquelle l'acide polycarboxylique ou son anhydride est choisi parmi, seul ou en mélange,
- les acides polycarboxyliques linéaires, ramifiés et/ou cycliques, saturés ou insaturés, voire aromatiques, comprenant 2 à 50 atomes de carbone; ledit acide comprenant au moins deux groupes carboxyliques COOH; et pouvant optionnellement comprendre 1 à 10 hétéroatomes, identiques ou différents, choisis parmi O, N et S; et/ou pouvant optionnellement comprendre au moins un radical perfluoré choisi parmi -CF₂- (divalent) ou -CF_{3;} et leurs anhydrides;
- les acides polycarboxyliques à chaîne saturée ou insaturée, linéaire ou ramifiée, comprenant au moins un hétéroatome choisi parmi O, N et/ou S, notamment 1 à 10 hétéroatomes identiques ou différents, et/ou comprenant au moins un radical perfluoré -CF₂- ou -CF₃ et possédant par ailleurs au moins 2 groupes carboxyliques COOH; et leurs anhydrides;
- les acides polycarboxyliques hétérocycliques, saturé ou insaturé, voire aromatique, comprenant au moins un hétéroatome choisi parmi O, N et/ou S, notamment 1 à 10 hétéroatomes identiques ou différents, et au moins 2 groupes carboxyliques COOH; et leurs anhydrides;
- les acides polycarboxyliques dérivés de sucre, susceptibles d'être obtenus notamment par oxydation d'un aldose, et comprenant au moins 2 groupes carboxyliques COOH; et leurs anhydrides;
- l'anhydride itaconique et le 1,4-monoanhydride de l'acide 1,4,5,8-naphthalenetetracarboxylique;
- les aminoacides polycarboxyliques (y compris hétérocycliques), c'est-à-dire les acides polycarboxyliques à chaîne saturée ou insaturée, linéaire, ramifiée, et/ou cyclique, comprenant éventuellement au moins un hétéroatome choisi parmi O, N et/ou S, notamment 1 à 10 hétéroatomes identiques ou différents, et/ou comprenant éventuellement au moins un radical perfluoré -CF₂- ou -CF₃; et comprenant en outre au moins une fonction amine primaire, secondaire ou tertiaire et possédant par ailleurs au moins 2 groupes carboxyliques COOH; et leurs anhydrides.

13. Composition selon l'une des revendications précédentes, dans laquelle l'acide polycarboxylique ou son anhydride est choisi parmi l'acide isophtalique ou l'anhydride phtalique.

14. Composition selon l'une des revendications précédentes, dans laquelle l'acide polycarboxylique et/ou son anhydride cyclique représente 5 à 60% en poids, notamment 10 à 40% en poids, et mieux 12 à 25% en poids, du poids total du polycondensat formant le squelette polymérique.

15. Composition selon l'une des revendications précédentes, dans laquelle l'acide polycarboxylique et/ou son anhydride cyclique représente 10 à 40% en poids, et mieux 12 à 25% en poids, du poids total du polycondensat formant le squelette polymérique.

16. Composition selon la revendication 10, dans laquelle dans le groupe de jonction porteur de groupes isocyanates, le radical A est de formule : 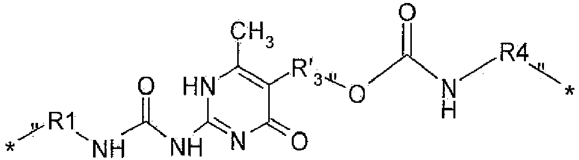 dans laquelle les radicaux R1, R3, R'3 et R4, indépendamment l'un de l'autre, sont choisis parmi les radicaux suivants : méthylène, 1,2-éthylène, 1,6-héxylène, 1,4-butylène, 1,6-(2,4,4-triméthylhexylène), 1,4-(4-méthylpentylène), 1,5-(5-méthylhexylène); 1,6-(6-méthylheptylène); 1,5-(2,2,5-triméthylhexylène), 1,7-(3,7-diméthyloctylène); 4,4'-méthylènebiscyclohexylène; 2-méthyl-1,3-phénylène; 4-méthyl-1,3-phénylène ; 4,4'-biphénylèneméthylène, 1,2-tolylène, 1,4-tolylène, 2,4-tolylène, 2,6-tolylène; 1,5-naphtylène; 4,4'-methylenebis(phényl); tétraméthyl xylylène; le radical divalent dérivé de l'isophorone.

17. Composition selon l'une des revendications précédentes, dans laquelle dans le groupe de jonction porteur de groupes isocyanates , le radical A est de formule

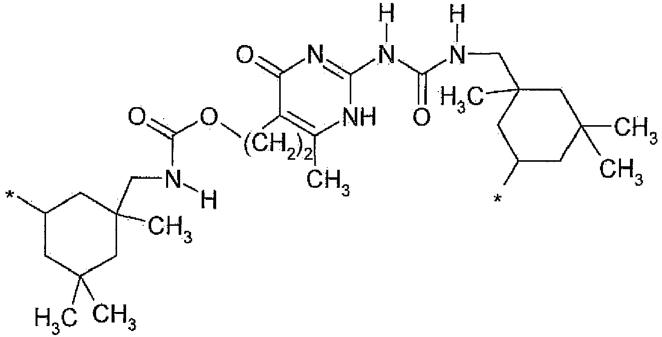

18. Composition selon l'une des revendications précédentes, dans laquelle dans le groupe de jonction porteur de groupes isocyanates, le radical B est de formule

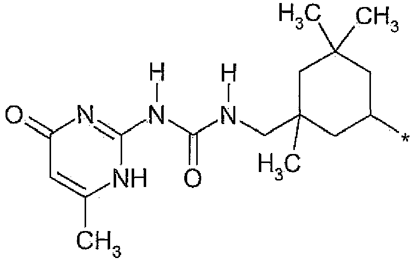

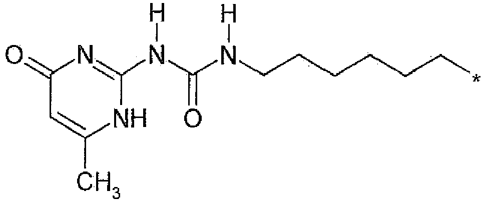

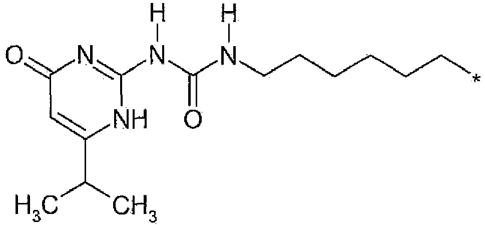

19. Composition selon l'une des revendications précédentes, dans laquelle le polycondensat est présent en une quantité comprise entre 0,1 et 80% en poids, de préférence entre 1 et 50% en poids, notamment entre 10 et 45% en poids, voire entre 20 et 40% en poids, et mieux entre 25 et 35% en poids, par rapport au poids de la composition cosmétique ou pharmaceutique finale.

20. Composition selon l'une des revendications précédentes, dans laquelle le milieu cosmétiquement ou dermatologiquement acceptable comprend au moins une phase grasse liquide, qui peut comprendre au moins un composé choisi parmi les huiles et/ou solvants d'origine minérale, animale, végétale ou synthétique, carbonés, hydrocarbonés, fluorés et/ou silicones, volatils ou non volatils, seuls ou en mélange.

21. Composition selon l'une des revendications précédentes, dans laquelle le milieu cosmétiquement ou dermatologiquement acceptable comprend au moins une huile et/ou un solvant choisi parmi, seul ou en mélange, l'isododécane, les polyisobutylènes hydrogénés, l'isononanoate d'isononyle, l'octyldodécanol, la phényl triméthicone, les benzoates d'alkyle en C12-C15, les acétates d'éthyle et de butyle, et/ou la D5 (décaméthylcyclopentasiloxane).

22. Composition selon l'une des revendications précédentes, se présentant sous la forme d'un produit de soin et/ou de maquillage de la peau du corps ou du visage, des lèvres, des cils, des sourcils, des cheveux, du cuir cheveu ou des ongles; d'un produit solaire ou autobronzant; d'un produit capillaire notamment de coloration, de conditionnement et/ou de soin des cheveux.

23. Procédé de traitement cosmétique des matières kératiniques, notamment de la peau du corps ou du visage, des lèvres, des ongles, des cheveux et/ou des cils, comprenant l'application sur lesdites matières d'une composition cosmétique telle que définie à l'une des revendications 1 à 22.

## Patentansprüche

1. Kosmetische oder dermatologische Zusammensetzung, umfassend in einem kosmetisch bzw. dermatologisch unbedenklichen Medium ein Polymer, das Folgendes umfasst:
(a) eine Polymerhauptkette, die durch Reaktion von:
- 10 bis 60 Gew. -%, bezogen auf das Gesamtgewicht der Hauptkette, mindestens eines Polyols mit 3 bis 6 Hydroxylgruppen;
- 0,1 bis 80 Gew.-%, bezogen auf das Gesamtgewicht der Hauptkette, einer Mischung einer aromatischen Monocarbonsäure mit 6 bis 32 Kohlenstoffatomen und einer nicht aromatischen Monocarbonsäure mit 6 bis 32 Kohlenstoffatomen;
- 5 bis 60 Gew.-%, bezogen auf das Gesamtgewicht der Hauptkette, mindestens einer Polycarbonsäure mit mindestens 2 Carboxylgruppen COOH und/oder eines cyclischen Anhydrids einer derartigen Polycarbonsäure
erhältlich ist; und
(b) mindestens eine Verbindungsgruppe, die an die Polymerhauptkette gebunden ist und zur Ausbildung von H-Brückenbindungen mit einer oder mehreren Partner-Verbindungsgruppen befähigt ist, wobei an jeder Paarung einer Verbindungsgruppe mindestens 3 H-Brückenbindungen (Wasserstoffbrückenbindungen) beteiligt sind,
wobei die Hauptkette über mindestens eine Urethanfunktion an die Verbindungsgruppe gebunden ist, wobei die Isocyanatgruppen tragende Verbindungsgruppe die Formel OCN-A-NCO, wobei der zweiwertige Rest A die folgende Struktur aufweist: 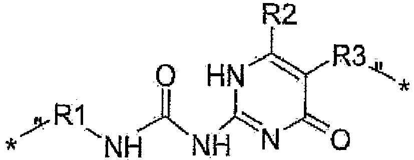 worin:
- R1 und R3 gleich oder verschieden sind und für einen zweiwertigen Kohlenstoffrest, der aus (i) einer linearen oder verzweigten C₁-C₃₀-Alkylgruppe, (ii) einer C₄-C₁₂-Cycloalkylgruppe und (iii) einer C₄-C₁₂-Arylgruppe, die gegebenenfalls 1 bis 8 aus O, N, S, F, Si und P ausgewählte Heteroatome umfasst und/oder gegebenenfalls durch eine Ester- oder Amidfunktion oder einen C₁-C₁₂-Alkylrest substituiert ist, ausgewählt ist; oder eine Mischung dieser Gruppen stehen;
- R2 für H, Halogen (Br, Cl, F) oder eine lineare, verzweigte oder cyclische, gesättigte oder ungesättigte, gegebenenfalls aromatische einwertige C₁-C₃₂-Kohlenwasserstoffgruppe, die ein oder mehrere aus O, S, N, P, F ausgewählte Heteroatome umfassen kann, steht;
oder die Formel B-NCO, wobei der einwertige Rest B die folgende Formel aufweist: 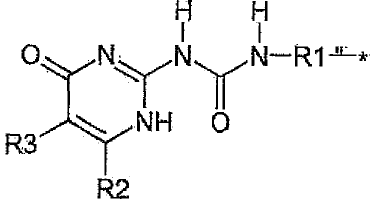 worin:
- der Rest R₁ für eine zweiwertige Kohlenstoffgruppe, die aus (i) einer linearen oder verzweigen C₁-C₃₀-Alkylgruppe, (ii) einer C₄-C₁₂-Cycloalkylgruppe und (iii) einer C₄-C₁₂-Arylgruppe, die gegebenenfalls 1 bis 8 aus O, N, S, F, Si und P ausgewählte Heteroatome umfasst und/oder gegebenenfalls durch eine Ester- oder Aminfunktion oder einen C₁-C₁₂-Alkylrest substituiert ist, ausgewählt ist; oder eine Mischung dieser Gruppen steht;
- die Reste R2 und R3 gleich oder verschieden sind und für ein Wasserstoffatom oder einen linearen, verzweigten oder cyclischen, gesättigten oder ungesättigten C1-C32-Kohlenstoffrest, insbesondere Kohlenwasserstoffrest (Alkylrest), der gegebenenfalls ein oder mehrere aus O, N, S, F, Si und P ausgewählte Heteroatome umfasst, stehen;
aufweist.

2. Zusammensetzung nach Anspruch 1, wobei das Polyol, alleine oder als Mischung, aus
- Triolen, wie 1,2,4-Butantriol, 1,2,6-Hexantriol, Trimethylolethan, Trimethylolpropan, Glycerin;
- Tetraolen, wie Pentaerythritol, Erythritol, Diglycerin oder Ditrimethylolpropan;
- Pentolen, wie Xylitol,
- Hexolen, wie Sorbitol und Mannitol oder auch Dipentaerythritol oder Triglycerin
ausgewählt ist.

3. Zusammensetzung nach einem der vorhergehenden Ansprüche, wobei es sich bei dem Polyol um Pentaerythritol handelt.

4. Zusammensetzung nach einem der vorhergehenden Ansprüche, wobei das Polyol bzw. die Mischung von Polyolen 10 bis 60 Gew.-%, insbesondere 12 bis 40 Gew.-% und noch besser 14 bis 25 Gew.-% des Gesamtgewichts des die Polymerhauptkette bildenden Polykondensats ausmacht.

5. Zusammensetzung nach einem der vorhergehenden Ansprüche, wobei das Polyol bzw. die Mischung von Polyolen 10 bis 25 Gew.-% des Gesamtgewichts des die Polymerhauptkette bildenden Polykondensats ausmacht.

6. Zusammensetzung nach einem der vorhergehenden Ansprüche; wobei die aromatische Monocarbonsäure, alleine oder als Mischung, aus:
Benzoesäure, o-Toluylsäure, m-Toluylsäure, p-Toluylsäure, 1-Naphthoesäure, 2-Naphthoesäure, 4-tert-Butylbenzoesäure, 1-Methyl-2-naphthoesäure und 2-Isopropyl-1-naphthoesäure
ausgewählt ist.

7. Zusammensetzung nach einem der vorhergehenden Ansprüche, wobei es sich bei der aromatischen Monocarbonsäure um Benzoesäure handelt.

8. Zusammensetzung nach einem der vorhergehenden Ansprüche, wobei die nicht aromatische Monocarbonsäure, alleine oder als Mischung, aus
- gesättigten Monocarbonsäuren wie Capronsäure, Caprylsäure, Isoheptansäure, 4-Ethylpentansäure, 2-Ethylhexansäure, 4,5-Dimethylhexansäure, 2-Heptylheptansäure, 3,5,5-Trimethylhexansäure, Octansäure, Isooctansäure, Nonansäure, Decansäure, Isononansäure, Laurinsäure, Tridecansäure, Myristinsäure, Palmitinsäure, Stearinsäure, Isostearinsäure, Arachidinsäure, Behensäure, Cerotinsäure (Hexacosansäure); Cyclopentancarbonsäure, Cyclopentanessigsäure, 3-Cyclopentylpropionsäure, Cyclohexancarbonsäure, Cyclohexylessigsäure and 4-Cyclohexylbuttersäure;
- nicht aromatischen ungesättigten Monocarbonsäuren, wie Caproleinsäure, Obtusilinsäure, Undecylensäure, Dodecylensäure, Linderinsäure, Myristoleinsäure, Physeterinsäure, Tsuzuinsäure, Palmitoleinsäure, Ölsäure, Petroselinsäure, Vaccensäure, Elaidinsäure, Gondoinsäure, Gadoleinsäure, Erucasäure, Cetoleinsäure, Nervonsäure, Ölsäure, Linolsäure, Linolensäure, Arachidonsäure, Rumensäure, Eicosapentaensäure und Docosahexaensäure;
- konjugierten Säuren wie Stillingasäure (10:2 2t,4c), Rumensäure (18:2, 9c, 11t), konjugierter Linolsäure (18:2 10t,12c), konjugierter Linolensäure (18:3 9c,11t,15c), konjugierter Linolensäure (18:3 6c,9c,11t) and alpha-Eleostearinsäure (18:3 9c,11t,13t), alleine oder als Mischung;
ausgewählt ist.

9. Zusammensetzung nach einem der vorhergehenden Ansprüche, wobei die nicht aromatische Monocarbonsäure aus Laurinsäure, Palmitinsäure, Stearinsäure, Behensäure, 2-Ethylhexansäure, Isooctansäure, Isononansäure, Isostearinsäure, Caproleinsäure, Obtusilinsäure, Undecylensäure, Dodecylensäure, Linderinsäure, Myristoleinsäure, Physeterinsäure, Tsuzuinsäure, Palmitoleinsäure, Ölsäure, Linolsäure, Linolensäure, Arachidonsäure und Mischungen davon ausgewählt ist.

10. Zusammensetzung nach einem der vorhergehenden Ansprüche, wobei die Mischung von Monocarbonsäuren 0,1 bis 80 Gew.-%, insbesondere 0,5 bis 70 Gew.-%, sogar 1 bis 65 Gew.-% und noch besser 1,5 bis 60 Gew.-% des Gesamtgewichts des die Polymerhauptkette bildenden Polykondensats ausmacht.

11. Zusammensetzung nach einem der vorhergehenden Ansprüche, wobei die Mischung von Monocarbonsäuren 1,5 bis 60 Gew.-% des Gesamtgewichts des die Polymerhauptkette bildenden Polykondensats ausmacht.

12. Zusammensetzung nach einem der vorhergehenden Ansprüche, wobei die Polycarbonsäure bzw. ihr Anhydrid, alleine oder als Mischung, aus
- linearen, verzweigten und/oder cyclischen, gesättigten oder ungesättigten oder sogar aromatischen Polycarbonsäuren mit 2 bis 50 Kohlenstoffatomen; wobei die Säure mindestens zwei Carboxylgruppen COOH umfasst und gegebenenfalls 1 bis 10 gleiche oder verschiedene, aus O, N und S ausgewählte Heteroatome umfassen kann und/oder gegebenenfalls mindestens einen perfluorierten Rest, der aus -CF₂- (zweiwertig) oder -CF₃ ausgewählt ist, umfassen kann; und Anhydriden davon;
- Polycarbonsäuren mit einer gesättigten oder ungesättigten, linearen oder verzweigten Kette mit mindestens einem aus O, N und/oder S ausgewählten Heteroatom, insbesondere 1 bis 10 gleichen oder verschiedenen Heteroatomen, und/oder mit mindestens einem perfluorierten Rest -CF₂- oder
- CF₃ und außerdem mit mindestens 2 Carboxylgruppen COOH und Anhydriden davon;
- gesättigten oder ungesättigten oder sogar aromatischen heterocyclischen Polycarbonsäuren mit mindestens einem aus O, N und/oder S ausgewählten Heteroatom, insbesondere 1 bis 10 gleichen oder verschiedenen Heteroatomen, und mindestens 2 Carboxylgruppen COOH und Anhydriden davon;
- von Zucker abgeleiteten Polycarbonsäuren, die insbesondere durch Oxidation einer Aldose erhältlich sind und mindestens 2 Carboxylgruppen COOH umfassen; und Anhydriden davon;
- Itaconsäureanhydrid und 1,4,5,8-Naphthalintetracarbonsäure-1,4-monoanhydrid;
- Aminopolycarbonsäuren (einschließlich heterocyclischer Aminopolycarbonsäuren), d.h. Polycarbonsäuren mit einer gesättigten oder ungesättigten, linearen, verzweigten und/oder cyclischen Kette, die gegebenenfalls mindestens ein aus O, N und/oder S ausgewähltes Heteroatom, insbesondere 1 bis 10 gleiche oder verschiedene Heteroatome, umfassen, und/oder gegebenenfalls mindestens einen perfluorierten Rest -CF₂- oder-CF₃ umfassen und außerdem mindestens eine primäre, sekundäre oder tertiäre Aminfunktion umfassen und darüber hinaus mindestens 2 Carboxylgruppen COOH umfassen; und Anhydriden davon
ausgewählt sind.

13. Zusammensetzung nach einem der vorhergehenden Ansprüche, wobei die Polycarbonsäure bzw. ihr Anhydrid aus Isophthalsäure oder Phthalsäureanhydrid ausgewählt ist.

14. Zusammensetzung nach einem der vorhergehenden Ansprüche, wobei die Polycarbonsäure und/oder ihr cyclisches Anhydrid 5 bis 60 Gew.-%, insbesondere 10 bis 40 Gew.-% und noch besser 12 bis 25 Gew.-% des Gesamtgewichts des die Polymerhauptkette bildenden Polykondensats ausmacht.

15. Zusammensetzung nach einem der vorhergehenden Ansprüche, wobei die Polycarbonsäure und/oder ihr cyclisches Anhydrid 10 bis 40 Gew.-% und noch besser 12 bis 25 Gew.-% des Gesamtgewichts des die Polymerhauptkette bildenden Polykondensats ausmacht.

16. Zusammensetzung nach Anspruch 10, wobei in der Isocyanatgruppen tragenden Verbindungsgruppe der Rest A die folgende Formel aufweist: 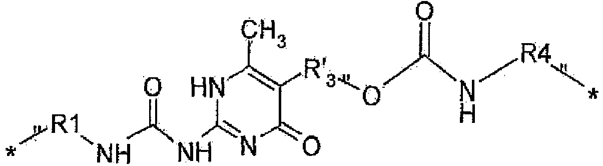 worin die Reste R1, R3, R'3 und R4 unabhängig voneinander aus den folgenden Resten ausgewählt sind: Methylen, 1,2-Ethylen, 1,6-Hexylen, 1,4-Butylen, 1,6-(2,4,4-Trimethylhexylen), 1,4-(4-Methylpentylen), 1,5-(5-Methylhexylen); 1,6-(6-Methylheptylen) ; 1,5-(2,2,5-Trimethylhexylen), 1,7-(3,7-Dimethyloctylen); 4,4'-Methylenbiscyclohexylen; 2-Methyl-1,3-phenylen; 4-Methyl-1,3-phenylen; 4,4'-Biphenylenmethylen, 1,2-Tolylen, 1,4-Tolylen, 2,4-Tolylen, 2,6-Tolylen; 1,5-Naphthylen; 4,4'-Methylenbis(phenyl); Tetramethylxylylen und dem von Isophoron abgeleiteten zweiwertigen Rest.

17. Zusammensetzung nach einem der vorhergehenden Ansprüche, wobei in der Isocyanatgruppen tragenden Verbindungsgruppe der Rest A die folgende Formel aufweist:

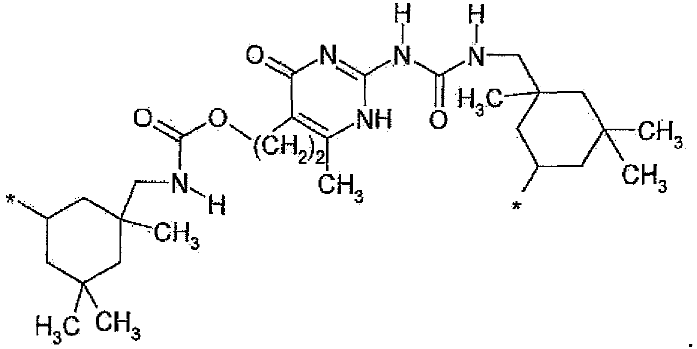

18. Zusammensetzung nach einem der vorhergehenden Ansprüche, wobei in der Isocyanatgruppen tragenden Verbindungsgruppe der Rest B die folgende Formel aufweist:

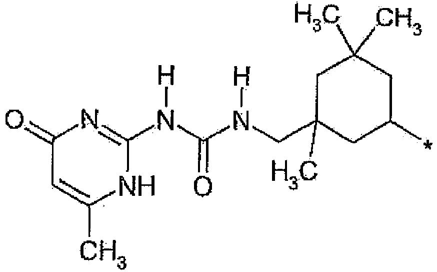

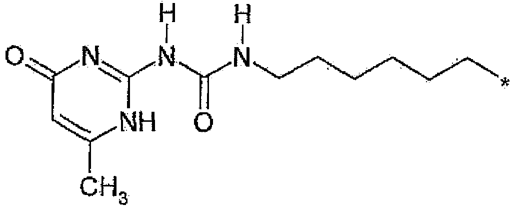

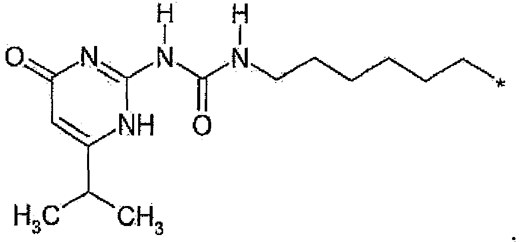

19. Zusammensetzung nach einem der vorhergehenden Ansprüche, wobei das Polykondensat in einer Menge zwischen 0,1 und 80 Gew.-%, vorzugsweise zwischen 1 und 50 Gew.-%, insbesondere zwischen 10 und 45 Gew.-%, sogar zwischen 20 und 40 Gew.-% und noch besser zwischen 25 und 35 Gew.-%, bezogen auf das Gewicht der fertigen kosmetischen oder pharmazeutischen Zusammensetzung, vorliegt.

20. Zusammensetzung nach einem der vorhergehenden Ansprüche, wobei das kosmetisch bzw. dermatologisch unbedenkliche Medium mindestens eine flüssige Fettphase umfasst, die mindestens eine aus Ölen und/oder Lösungsmitteln mineralischer, tierischer, pflanzlicher oder synthetischer Herkunft, die kohlenstoffhaltig, kohlenwasserstoffhaltig, fluorhaltig und/oder silikonhaltig sind und flüchtig oder nichtflüchtig sind, alleine oder als Mischung, ausgewählte Verbindung umfassen kann.

21. Zusammensetzung nach einem der vorhergehenden Ansprüche, wobei das kosmetisch bzw. dermatologisch unbedenkliche Medium mindestens ein Öl und/oder ein Lösungsmittel umfasst, das, alleine oder als Mischung, aus Isododecan, hydrierten Polyisobutylenen, Isononylisononanoat, Octyldodecanol, Phenyltrimethicon, Benzoesäure-C12-C15-alkylestern, Essigsäureethylestern, Essigsäurebutylestern und/oder D5 (Decamethylcyclopentasiloxan) ausgewählt ist.

22. Zusammensetzung nach einem der vorhergehenden Ansprüche, die in Form eines Produkts zur Pflege und/oder zum Make-up der Körper- oder Gesichtshaut, der Lippen, der Wimpern, der Augenbrauen, der Haare, der Kopfhaut oder der Nägel, eines Sonnenschutz- oder Selbstbräunungsprodukts oder eines Haarprodukts, insbesondere zum Färben, Konditionieren und/oder Pflegen der Haare, vorliegt.

23. Verfahren zur kosmetischen Behandlung von Keratinmaterialien, insbesondere der Körper- oder Gesichtshaut, der Lippen, der Nägel, der Haare und/oder der Wimpern, bei dem man auf die Materialien eine kosmetische Zusammensetzung gemäß einem der Ansprüche 1 bis 22 aufbringt.

## Claims

1. Cosmetic or dermatological composition comprising, in a cosmetically or dermatologically acceptable medium, a polymer comprising:
(a) a polymer backbone that may be obtained by reacting:
- from 10% to 60% by weight, relative to the total weight of the said backbone, of at least one polyol comprising 3 to 6 hydroxyl groups;
- from 0.1% to 80% by weight, relative to the total weight of the said backbone, of a mixture of aromatic monocarboxylic acid containing 6 to 32 carbon atoms and of non aromatic monocarboxylic acid containing 6 to 32 carbon atoms;
- from 5% to 60% by weight, relative to the total weight of the said backbone, of at least one polycarboxylic acid comprising at least two carboxylic groups COOH, and/or a cyclic anhydride of such a polycarboxylic acid;
and
- (b) at least one junction group linked to the said polymer backbone and capable of establishing H bonds with one or more partner junction groups, each pairing of a junction group involving at least three H (hydrogen) bonds,
said backbone being linked to the junction group by at least one urethane function,
the junction group bearing isocyanate groups being of formula OCN-A-NCO, the divalent radical A having the structure: 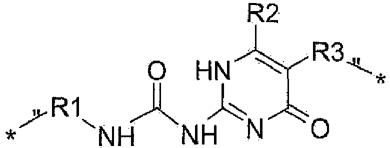 in which:
- R1 and R3, which may be identical or different, represent a divalent carbon-based radical chosen from (i) a linear or branched C₁-C₃₀ alkyl group, (ii) a C₄-C₁₂ cycloalkyl group and (iii) a C₄-C₁₂ aryl group; optionally comprising 1 to 8 heteroatoms chosen from O, N, S, F, Si and P; and/or optionally substituted with an ester or amide function or with a C₁-C₁₂ alkyl radical; or a mixture of these groups;
- R2 represents H, halogen (Br, Cl or F) or a linear, branched or cyclic, saturated or unsaturated, optionally aromatic C₁-C₃₂ monovalent hydrocarbon-based group, which may contain one or more heteroatoms chosen from O, S, N, P and F,
or of formula B-NCO, the monovalent radical B having the formula: 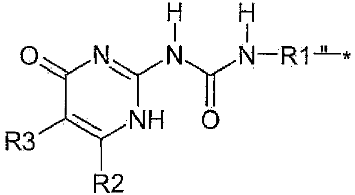 in which:
- the radical R1 represents a divalent carbon-based group chosen from (i) a linear or branched C₁-C₃₀ alkyl group, (ii) a C₄-C₁₂ cycloalkyl group and (iii) a C₄-C₁₂ aryl group; optionally comprising 1 to 8 heteroatoms chosen from O, N, S, F, Si and P; and/or optionally substituted with an ester or amide function or with a C₁-C₁₂ alkyl radical; or a mixture of these groups;
- the radicals R2 and R3, which may be identical or different, represent a hydrogen atom or a linear, branched or cyclic, saturated or unsaturated C₁-C₃₂ carbon-based, especially hydrocarbon-based (alkyl), radical, which may comprise one or more heteroatoms chosen from O, N, S, F, Si and P.

2. Composition according to Claim 1, in which the polyol is chosen, alone or as a mixture, from:
- triols, such as 1,2,4-butanetriol, 1,2,6-hexanetriol, trimethylolethane, trimethylolpropane or glycerol;
- tetraols, such as pentaerythritol, erythritol, diglycerol or ditrimethylolpropane;
- pentols such as xylitol,
- hexols such as sorbitol and mannitol; or alternatively dipentaerythritol or triglycerol.

3. Composition according to one of the preceding claims, in which the polyol is pentaerythritol.

4. Composition according to one of the preceding claims, in which the polyol, or the polyol mixture, represents 10% to 60% by weight, especially 12% to 40% by weight and better still 14% to 25% by weight relative to the total weight of the polycondensate forming the polymer backbone.

5. Composition according to one of the preceding claims, in which the polyol, or the polyol mixture, represents 10% to 25% by weight relative to the total weight of the polycondensate forming the polymer backbone.

6. Composition according to one of the preceding claims, in which the aromatic monocarboxylic acid is chosen, alone or as a mixture, from:
benzoic acid, o-toluic acid, m-toluic acid, p-toluic acid, 1-naphthoic acid, 2-naphthoic acid, 4-tert-butylbenzoic acid, 1-methyl-2-naphthoic acid and 2-isopropyl-1-naphthoic acid.

7. Composition according to one of the preceding claims, in which the aromatic monocarboxylic acid is benzoic acid.

8. Composition according to one of the preceding claims, in which the non aromatic monocarboxylic acid is chosen, alone or as a mixture, from:
- saturated monocarboxylic acids such as caproic acid, caprylic acid, isoheptanoic acid, 4-ethylpentanoic acid, 2-ethylhexanoic acid, 4,5-dimethylhexanoic acid, 2-heptylheptanoic acid, 3,5,5-trimethylhexanoic acid, octanoic acid, isooctanoic acid, nonanoic acid, decanoic acid, isononanoic acid, lauric acid, tridecanoic acid, myristic acid, palmitic acid, stearic acid, isostearic acid, arachidic acid, behenic acid, cerotic acid (hexacosanoic acid); cyclopentane-carboxylic acid, cyclopentaneacetic acid, 3-cyclopentylpropionic acid, cyclohexanecarboxylic acid, cyclohexylacetic acid and 4-cyclohexylbutyric acid;
- non-aromatic unsaturated monocarboxylic acids, such as caproleic acid, obtusilic acid, undecylenic acid, dodecylenic acid, linderic acid, myristoleic acid, physeteric acid, tsuzuic acid, palmitoleic acid, oleic acid, petroselinic acid, vaccenic acid, elaidic acid, gondoic acid, gadoleic acid, erucic acid, cetoleic acid, nervonic acid, oleic acid, linoleic acid, linolenic acid, arachidonic acid, rumenic acid, eicosapentaenoic acid and docosahexaenoic acid;
- conjugated acids such as stillinguic acid (10:2 2t,4c), rumenic acid (18:2 9c,11t), conjugated linoleic acid (18:2 10t,12c), conjugated linolenic acid (18:3 9c,11t,15c), conjugated linolenic acid (18:3 6c,9c,11t) and alpha-eleostearic acid (18:3 9c,11t,13t), alone or as a mixture.

9. Compositions according to one of the preceding clams, in which the non aromatic monocarboxylic acid is chosen from lauric acid, palmitic acid, stearic acid, behenic acid, 2-ethylhexanoic acid, isooctanoic acid, isononanoic acid, isostearic acid, benzoic acid, 4-tert-butylbenzoic acid, o-toluic acid, m-toluic acid, 1-naphthoic acid, caproleic acid, obtusilic acid, undecylenic acid, dodecylenic acid, linderic acid, myristoleic acid, physeteric acid, tsuzuic acid, palmitoleic acid, oleic acid, linoleic acid, linolenic acid or arachidonic acid, and mixtures thereof.

10. Composition according to one of the preceding claims, in which the mixture of monocarboxylic acids represents 0.1% to 80% by weight, especially 0.5% to 70% by weight, or even 1% to 65% by weight, and better still 1.5% to 60% by weight, relative to the total weight of the polycondensate forming the polymer backbone.

11. Composition according to one of the preceding claims, in which the mixture of monocarboxylic acids represents 1.5% to 60% by weight, relative to the total weight of the polycondensate forming the polymer backbone.

12. Composition according to one of the preceding claims, in which the polycarboxylic acid or the anhydride thereof is chosen, alone or as a mixture, from:
- linear, branched and/or cyclic, saturated or unsaturated, or even aromatic, polycarboxylic acids containing 2 to 50 carbon atoms; the said acid comprising at least two carboxylic groups COOH; and optionally possibly comprising 1 to 10 identical or different heteroatoms chosen from 0, N and S; and/or optionally possibly comprising at least one perfluoro radical chosen from -CF₂- (divalent) and -CF₃; and anhydrides thereof;
- polycarboxylic acids with a saturated or unsaturated, linear or branched chain comprising at least one heteroatom chosen from O, N and/or S, especially 1 to 10 identical or different heteroatoms, and/or comprising at least one perfluoro radical -CF₂- or -CF₃ and moreover containing at least two carboxylic groups COOH; and anhydrides thereof;
- saturated or unsaturated, or even aromatic, heterocyclic polycarboxylic acids, comprising at least one heteroatom chosen from 0, N and/or S, especially 1 to 10 identical or different heteroatoms, and at least two carboxylic groups COOH; and anhydrides thereof;
- sugar-based polycarboxylic acids, which may be obtained especially by oxidation of an aldose, and comprising at least two carboxylic groups COOH; and anhydrides thereof;
- itaconic anhydride and 1,4,5,8-naphthalenetetra-carboxylic acid 1,4-monoanhydride;
- polycarboxylic (including heterocyclic) amino acids, i.e. polycarboxylic acids containing a saturated or unsaturated, linear, branched and/or cyclic chain, optionally comprising at least one heteroatom chosen from O, N and/or S, especially 1 to 10 identical or different heteroatoms, and/or optionally comprising at least one perfluoro radical -CF₂- or -CF₃; and also comprising at least one primary, secondary or tertiary amine function and moreover containing at least two carboxylic groups COOH; and anhydrides thereof.

13. Composition according to one of the preceding claims, in which the polycarboxylic acid, or the anhydride thereof, is chosen from isophthalic acid and phthalic anhydride.

14. Composition according to one of the preceding claims, in which the polycarboxylic acid and/or the cyclic anhydride thereof represents 5% to 60% by weight, especially 10% to 40% by weight and better still 12% to 25% by weight relative to the total weight of the polycondensate forming the polymer backbone.

15. Composition according to one of the preceding claims, in which the polycarboxylic acid and/or the cyclic anhydride thereof represents 10% to 40% by weight and better still 12% to 25% by weight relative to the total weight of the polycondensate forming the polymer backbone.

16. Composition according to Claim 10, in which, in the junction group bearing isocyanate groups, the radical A has the formula: 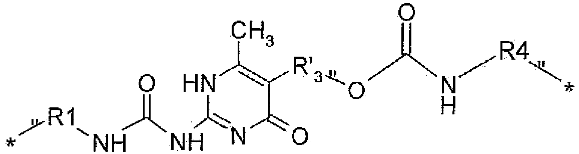 in which the radicals R1, R3, R'3 and R4, independently of each other, are chosen from the following radicals: methylene, 1,2-ethylene, 1,6-hexylene, 1,4-butylene, 1,6-(2,4,4-trimethylhexylene), 1,4-(4-methylpentylene), 1,5-(5-methylhexylene); 1,6-(6-methylheptylene); 1,5-(2,2,5-trimethylhexylene), 1,7-(3,7-dimethyloctylene); 4,4'-methylenebiscyclohexylene; 2-methyl-1,3-phenylene; 4-methyl-1,3-phenylene; 4,4'-biphenylenemethylene, 1,2-tolylene, 1,4-tolylene, 2,4-tolylene, 2,6-tolylene; 1,5-naphthylene; 4,4'-methylenebis(phenyl); tetramethylxylylene; the divalent radical derived from isophorone.

17. Composition according to one of the preceding claims, in which, in the junction group bearing isocyanate groups, the radical A has the formula:

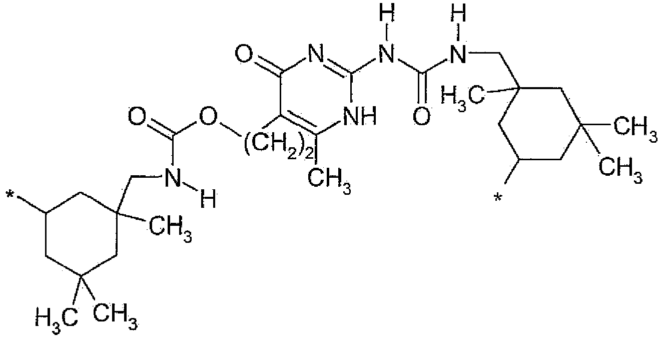

18. Composition according to one of the preceding claims, in which, in the junction group bearing isocyanate groups, the radical B has the formula:

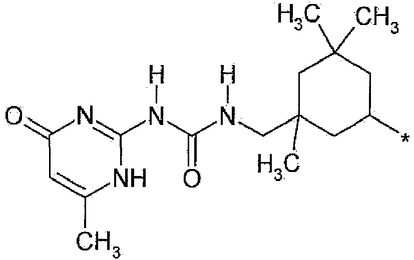

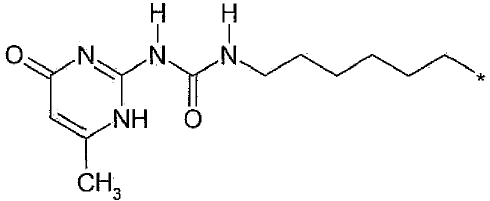

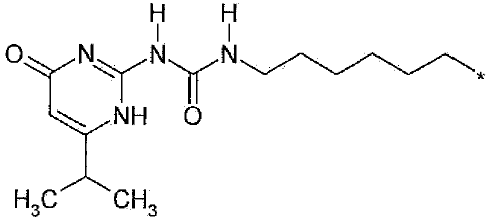

19. Composition according to one of the preceding claims, in which the polycondensate is present in an amount of between 0.1% and 80% by weight, preferably between 1% and 50% by weight, especially between 10% and 45% by weight, or even between 20% and 40% by weight, and better still between 25% and 35% by weight, relative to the weight of the final cosmetic or pharmaceutical composition.

20. Composition according to one of the preceding claims, in which the cosmetically or dermatologically acceptable medium comprises at least one liquid fatty phase, which may comprise at least one compound chosen from volatile or non-volatile carbon-based, hydrocarbon-based, fluoro and/or silicone oils and/or solvents of mineral, animal, plant or synthetic origin, alone or as a mixture.

21. Composition according to one of the preceding claims, in which the cosmetically or dermatologically acceptable medium comprises at least one oil and/or one solvent chosen, alone or as a mixture, from isododecane, hydrogenated polyisobutylenes, isononyl isononanoate, octyldodecanol, phenyl trimethicone, C₁₂-C₁₅ alkyl benzoates, ethyl and butyl acetates, and/or D5 (decamethylcyclopentasiloxane).

22. Composition according to one of the preceding claims, which is in the form of a product for caring for and/or making up bodily or facial skin, the lips, the eyelashes, the eyebrows, the hair, the scalp or the nails; an antisun or self-tanning product; a haircare product especially for dyeing, conditioning and/or caring for the hair.

23. Cosmetic process for treating keratin materials, especially bodily or facial skin, the lips, the nails, the hair and/or the eyelashes, comprising the application to the said materials of a cosmetic composition as defined in one of Claims 1 to 22.
